# EUROPEAN PATENT APPLICATION

(11) **EP 4 729 126 A1**
(43) Date of publication of application: **22.04.2026**
(21) Application number: 24207224.7
(22) Date of filing: 17.10.2024
(51) Int. Cl.: A61P 35/00, A61K 35/17, C07K 16/28

(54) **NK CELL-DERIVED EXTRACELLULAR VESICLES ARMED WITH MODIFIED MONOCLONAL ANTIBODIES TO TARGET CANCER CELLS, AND USE THEREOF**

(71) Applicant: Centre National de la Recherche Scientifique, 75016 Paris (FR); Institut National de la Santé et de la Recherche Médicale (INSERM), 75013 Paris (FR); Universite de Montpellier (UM), 34090 Montpellier (FR)
(72) Inventor: VILLALBA GONZALEZ, Martín, 34070 Montpellier (FR); CAMPOS MORA, Mauricio, 34000 Montpellier (FR)
(74) Representative: Ungria López, Javier

(57) **Abstract**

The invention relates to NK cell-derived extracellular vesicles, as attractive candidates for new anti-tumor therapies. Natural killer (NK) cells participate in the anti-tumor immune response by recognizing and exerting cytolytic functions on target cells. Research has focused on taking advantage of these characteristics to generate NK cell-based immunotherapies to complement treatments with therapeutic monoclonal antibodies. However, in some environments, such as the tumor microenvironment, NK cells partly lose their cytotoxic ability limiting their clinical efficacy. The use of the active NK cell "parts" hereby claimed avoids the negative effect of the tumor microenvironment. The EV is assembled with an antibody that recognizes target cells. Being able to ensemble different antibodies makes this strategy very versatile, and allows the therapy to be used for the treatment of not only tumors but also to treat infections or autoimmune diseases among others.

## Description

### TECHNICAL FIELD

The present invention relates to the field of therapeutic treatment, particularly to natural killer (NK) cell-based immunotherapy. More particularly, the invention relates to a pharmaceutical composition comprising a CD16+ cell-derived extracellular vesicle (EV) that can be complexed with a recombinant polypeptide comprising a modified Fc region, in particular a modified CH2 domain, and a ligand binding domain. This recombinant polypeptide can direct and target the EV to a specific cell that is expressing a cell-surface marker (e.g., an antigen for the antibody) that can be recognized and bound by the antibody.

### BACKGROUND OF THE INVENTION

NK cells are vital components of the innate immune system, with cytolytic properties, and they perform an essential role in the immune surveillance for altered, damaged or transformed cells. Unlike CD8⁺ T cells and other adaptive immune cell types, NK cell receptors are germline-encoded and do not undergo rearrangement, nor do they display specificity. NK cells are not restricted to major histocompatibility complex recognition, and their activation requires multiple receptors. Indeed, their activation relies on finely tuning inhibitory and activating signals (Wu F, et al. Front Immunol. 2021; Wang X, et al. Cancers Basel. 2022).

NK cells are characterized in humans by expression of the surface marker CD56 in the absence of CD3, and circulate through the organism sensing inflammation signals. Upon encounter with target cells, NK cells recognize activation ligands and stress markers in target's surface, and trigger multiple mechanisms to eliminate target cells, which induces the release of cytoplasmic granules loaded with perforin and granzyme B within the immunological synapse, thereby inducing the lysis of target cells. Upon NK cell activation, CD107a expression is upregulated and it is positively correlated with cytokine secretion and cytotoxic activity.

NK cell-mediated anti-tumor activity is regulated through a repertoire of activating and inhibitory cell surface receptors. Activating receptors of NK cells include CD16, along with natural cytotoxicity receptor family members (NKp30, NKp44, NKp46), and co-stimulatory receptors such as LFA-1, 2B4, and 4-1BB. Upon activation, NK cells release cytotoxic granules containing perforin and granzymes to directly lyse tumor cells. CD16a is the most potent activating receptor expressed by NK cells. Of note, CD16a is the only receptor that can activate NK cells on its own, without any additional activation through other receptors. Accordingly, the Fc regions of IgG antibodies on opsonized cells serve to crosslink CD16a molecules and thus activate NK cells through a process termed antibody-dependent cell-mediated cytotoxicity (ADCC). To add more, NK cells can exert their cytotoxic potential through the expression of crucial death ligands, including Fas ligand (FasL) and tumor necrosis factor (TNF)-related apoptosis-inducing ligand (TRAIL), leading to target cell apoptosis after interacting with their receptor (Meng F, et al. J Hematol Oncol. 2023; Coënon L, Villalba M. Front Immunol. 2022).

These characteristics have made NK cells an attractive focus of research aimed to activation and expansion improvement, with the objective of therapeutic application in cancer. The combination therapy of NK cells expressing the FcγRIIIa (CD16) with adoptive immunotherapy of monoclonal antibodies (mAb), such as the anti-CD20 Rituximab (RTX), the anti-HER2 Trastuzumab, or the anti-EGFR Cetuximab (CTX), has important benefits that consist in helping antibody-dependent cellular cytotoxicity (ADCC), which is key in the clinical efficacy of these therapeutic antibodies.

For instance, WO2022023581A1 discloses a pharmaceutical composition comprising either a CD16+ cell, a NK cell, or a NK cell precursor, which are combined with a recombinant polypeptide that is capable of binding to the FcγRIII (CD16) surface protein. The recombinant polypeptide comprises a modified CH2 domain and a ligand binding domain. The modified CH2 domain through which the recombinant polypeptide binds the CD16 surface protein in a non-covalently fashion, has a demonstrated increase in C1q binding affinity due to specifics amino acid substitutions, such as S239D, I332E, H268F, S324T, G236A and A330L. The ligand binding domain comprises a sequence capable of binding to a target ligand, preferentially a ligand binding domain of any of the already known antibodies that are currently used to treat diseases or are yet developing. The composition as defined in the current patent application may be for use in a method for treating or preventing a cancer, an autoimmune disease, or an infectious disease in an individual in need thereof.

It is important to point out that molecules at the cell surface not only mediate intercellular interactions but also reveal characteristics fundamental to the lineage and function of that cell. These molecules are often also critical to cell function. A good example of this is the protein FcγRllla/CD16a, which is expressed on NK cells and binds immunoglobulin G (IgG), triggering a protective immune response. IgG1 from serum is likely the predominant ligand for CD16a under normal conditions.

It must be taken into account that every individual protein at the cell surface is potentially unique because of the compositional variability of post-translational modifications (PTMs) at multiple sites that are not directly encoded in the genome. Further, composition of the PTMs impacts protein function and possibly immune cell response including CD16a on NK cells. Little is known about CD16a processing, though composition impacted the affinity for IgG1 Fc in vitro. Presenting minimally remodeled N-glycans at N162 increased affinity by up to 50-fold for IgG1 Fc. It has been shown that N45 glycosylation also contributes to affinity by stabilizing CD16a structure; However, roles for the three remaining N-glycans in the CD16 structure remain undefined. Thus, there is a possibility that CD16a PTM composition impacts antibody binding affinity in vivo (Patel, et al. Mol Cell Proteomics. 2019).

PTM composition cannot be predicted from a DNA sequence. The predominant mammalian PTM, asparagine-linked (N-)glycosylation, includes two principal phases: the assembly of a lipid-linked oligosaccharide (LLO) and the transfer of the oligosaccharide to selected asparagine residues of polypeptide chains. Biosynthesis of the LLO takes place at both sides of the endoplasmic reticulum (ER) membrane and it involves a series of specific glycosyltransferases that catalyze the assembly of the branched oligosaccharide in a highly defined way. The functional receptor-binding unit of IgG1 is the Fc, a dimer that binds asymmetrically to CD16a. This asymmetric mode will bind to one of the two Fc chains providing the greatest affinity (Breitling, et al. Cold Spring Harb Perspect Biol. 2013; Patel, et al. Mol Cell Proteomics. 2019).

The generation of allogeneic expanded NK (eNK) cells with high cytotoxic function, and the elicit of strong ADCC responses mediated by NK cells armed with therapeutic mAbs have been a promising strategy for improving antitumor response and response rate. However, adoptive transfer of allogeneic NK cells to solid tumor patients have proved not really successful in several clinical trials. Challenges include difficulty in ex vivo expansion meeting clinical grade, tumor immune escape, limited in vivo persistence, and limited infiltration into solid tumors. And what is more, the tumor microenvironment (TME) inhibits NK cell responses. These factors directly hinder or limit the use of NK cells in solid tumor therapy.

EVs are highly heterogeneous membrane-bound carriers of various cargoes, including proteins, lipids, and nucleic acids. They are secreted by various cell types under both normal and pathological conditions, participate in intercellular communication, and influence various physiological and pathological processes. EVs are commonly classified as exosomes, microvesicles, or apoptotic bodies according to the mechanism of production. The three types of EVs differ in their size, origin, and mechanism of formation, but they all serve as means of communication between cells. In addition, they carry various cargoes that reflect their cells of origin and can be delivered to other cells, influencing their function (Sheta M, et al. Biology Basel. 2023; He S, et al. Front Immunol. 2024).

Exosomes are the smallest type of EV and are typically 30-150 nanometers in diameter. They originate from the endosomal system within cells. Exosomes contain a variety of molecular constituents, including proteins and RNA, from their cells of origin. Exosome can be taken up by neighboring or distant cells and influence their function. Microvesicles, also known as ectosomes, shedding vesicles, or microparticles, are larger than exosomes and have a diameter of -100-1000 nanometers. They are formed by direct outward budding and fission of the plasma membrane. Like exosomes, microvesicles contain a variety of molecules from their cells of origin and can influence other cells by fusing with their membranes or by being internalized. Apoptotic bodies are the largest type of EV: they are usually 1-5 micrometers in diameter and are generated during apoptosis, a type of programmed cell death. The disassembling cell body forms blebs that then break off to form apoptotic bodies that contain various cellular components, including organelles and DNA. Apoptotic bodies are usually taken up and digested by nearby phagocytic cells and can influence the immune response.

For several reasons, EVs have gained much attention in scientific research as next-generation carriers for drug delivery (Stenger T, et al. Front Immunol. 2024; Page A, et al. Cell Mol Immunol. 2024; Lugini L, et al. J Immunol. 2012; Federici C, et al. Front Immunol. 2020). Compared to synthetic nanoparticles, EVs have shown lower toxicity and immunogenicity in preclinical studies, reducing the risk of causing adverse reactions or immune responses. EVs are derived from cells and are formed by lipid bilayers, and this composition enhances its biocompatibility and stability allowing them to protect cargo molecules from degradation and harsh conditions in the body, such as enzymatic degradation and low-pH environments. It has been reported that EVs can reach their target organs after they travel in the bloodstream, and they can even deliver drugs by penetrating the blood-brain barrier (BBB). By engineering EVs to carry specific cargo molecules, such as therapeutic proteins, small interfering Ribonucleic Acid (RNAs) (siRNAs), or anti-cancer drugs, researchers can enhance drug stability and improve their targeted delivery to specific cells or tissues. This approach has the potential to minimize off-target effects and increase therapeutic efficacy, offering a more precise and effective treatment strategy (Claridge B, et al. Front Cell Dev Biol. 2021; Rafieezadeh D, et al. Int J Physiol Pathophysiol Pharmacol. 2024).

Immune cell-derived EVs and their use in antitumor therapy have been receiving some attention in recent years, with many studies confirming their great potential. Among them, NK cell-derived EVs (NK-EVs) have gained more attention for their unique biological properties. NK-EVs possess NK cell surface receptors and cytotoxic proteins enabling them to function similarly to parental cells. Unlike cells, nanoscale NK-EVs can easily diffuse and infiltrate solid tumors and own natural targeting and biocompatibility properties.

The patent publication WO2024072052A1 discloses an extracellular vesicle derived from NK cells on which cytokines and antibodies are expressed on the surface. The cells producing the EVs are transformed with the vectors that codify the cytokine and antibody of interest. Then, the EVs are isolated from the culture medium in which cells were transformed with the vectors. By expressing IL-15 and a cancer cell-specific antibody on the surface of human NK cells, it is possible to provide an EV that can exhibit strong cytotoxic activity specifically against cancer cells. The extracellular vesicle can be provided as an anticancer agent that is safe for the human body and exhibits significantly increased cytotoxicity for killing cancer cells.

Despite the number of studies demonstrating the efficacy of NK-EVs in cancer therapy, the development of therapeutical NK-EVs still faces significant challenges (Claridge B, et al. Front Cell Dev Biol. 2021; Qi Y, et. Front Bioeng Biotechnol. 2023; Chan A, et al. Int J Mol Sci. 2023). The first is the heterogeneity of NK-EVs, which complicates quality control and hinders a comprehensive understanding of their function. The main reason comes from the cell source and isolation methods for the production of NK-EVs. The optimal cell source and isolation method for EVs is still under investigation, with current methods facing challenges for improving the separation purity.

A second challenge would be selecting designs that improve the cycling stability and the cytotoxicity of NK-EVs. Although various endogenous and exogenous modification methods are used, there is no effective method to improve the loading efficiency of bioactive molecules without compromising the integrity of EVs, and most modification methods may cause clustering. Furthermore, it is necessary to evaluate the need for these modifications and their improvement in therapeutic efficacy. A reliable method to determine whether the loaded EVs contain active molecules is still urgently needed.

Specific, targeted delivery presents a major challenge for EV-based therapeutics. Sophisticated solutions are required to minimize off-target accumulation and maximize efficacy through the targeted transport and delivery of cargo. Additionally, different cell types and phenotypes internalize and traffic nano-carriers differently. This is especially relevant for EV therapeutics, as the heterogeneity of EVs can contribute to differential recognition and binding specificity and target cell uptake. Characterization of such mechanisms will be essential in the field's pursuit of accurate, potent, on-target delivery and uptake of EV therapeutics.

Finally, there is no uniform standard on the optimal NK-EV concentration, or how long it will take to achieve the desired therapeutic effect, as well as which delivery method and treatment regimen should be employed during treatment to achieve improved clinical outcomes. Although it is promising that in most studies NK-EVs did not show significant cytotoxicity to healthy cells even after 48 h of exposure, NK-EVs are known to be taken up by healthy cells and have shown cytotoxic effects in activated PBMCs, suggesting that they could have systemic side effects. Therefore, it is imperative to improve the targeting ability of NK-EVs. An object of the present invention is to fulfill all or part of these needs.

With the aim of overcoming these drawbacks in the technical field, the present invention is provided.

### DESCRIPTION OF THE INVENTION

### General description

The invention provides a product, a method for obtaining said product, and a pharmaceutical composition comprising the claimed product.

Based on the experimental evidence provided, the present invention discloses CD16+ EVs, which are combined efficiently and in a stable manner with a recombinant polypeptide comprising a modified Fc region. The EVs may in particular be derivative from the Natural Killer cell lineage. It has been demonstrated that a recombinant polypeptide as disclosed herein has a high affinity for the CD16 receptor for a long period of time.

This novel combination of the EVs complexed to the recombinant polypeptide, also called "armed EVs" hereinafter, is an effective way to improve the target activity of the NK-EVs by increasing the specificity towards target cells, and avoiding to be taken up by healthy cells. Arming EVs with molecules that recognize specific tumor receptors is also a good strategy to overcome the heterogeneity presented by tumor cells. For the first time, the target activity of the NK-EVs is directed by a recombinant polypeptide that is not expressed on the EV surface. This recombinant polypeptide comprises (i) a modified Fc region, preferably with at least one modification in a CH2 domain, and (ii) a ligand binding domain. In a preferred embodiment described herein, the recombinant polypeptide is an antibody with modifications in the CH2 domain which can be symmetrical, or asymmetrical, with respect to the pair of CH2 domains (or the pair of heavy chains) constituting the antibody. The recombinant polypeptide is capable of binding to the FcγRIII (CD16) surface protein in a non-covalent fashion through its modified Fc region. The ligand binding domain of the recombinant polypeptide refers to a sequence capable of binding to a target ligand, such as a target substance, a target compound, a target molecule or even a target cell, (i) for example a ligand binding domain which is capable of binding to a specific antigen of a target cell, or a sequence capable of binding to a natural or tumor antigen, such as the antigen binding domain of an antibody, which is capable of binding to said target or (ii) for example a ligand binding domain which is capable of binding to a cell receptor or binding to the antigen binding domain of an antibody, in which case the ligand binding domain comprises, or consists of, an antigen recognized by the antigen binding domain of said antibodies. This novel approach of combining EVs and recombinant polypeptides/antibodies resulting in armed EVs, facilitate the EV binding to target cells, and exhibits stronger binding capabilities and more specific cytotoxic capacity against the target cells. This new strategy is shown in the current study to be efficacious solving the unexpected cytotoxicity issues that are faced by EVs in current ongoing studies.

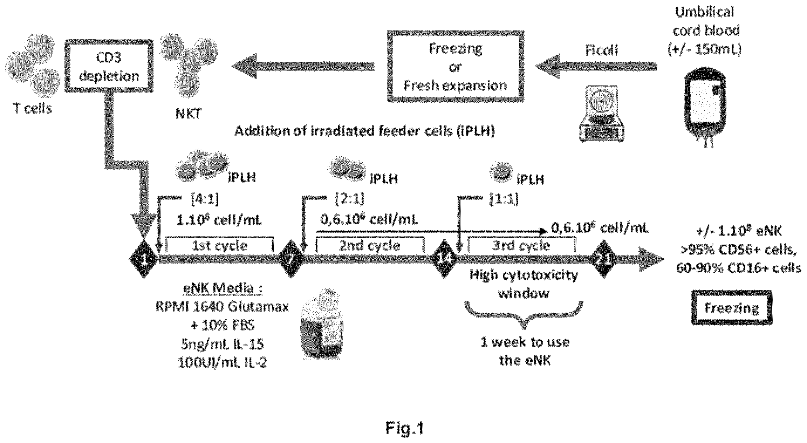

The innovative combination of the NK-EVs with the recombinant polypeptides disclosed in this patent, is only possible due to the fact that the NK-EVs are CD16+. Of note, previous scientific articles showed that despite CD16 membrane protein being present on the surface NK cells, NK-derived EVs did not contain CD16 (Federici C, et al. Front Immunol. 2020; Lugini L, et al. J Immunol. 2012). It was unexpected for the authors to get NK-EVs with CD16 expression, most likely due to their unique method to obtain NK-derived EVs and that is described herein. This recent and unexpected finding propelled the development of a new strategy to enrich EVs with antibodies that increases EV specificity and maximizes EV directed cytotoxicity towards the target cell of interest, resulting in a better therapeutic efficacy. Figure 1 represents the protocol followed to isolate and expand human NK cells. To functionally profile NK cell populations a sensitive and easy-to-use protocol is paramount. In the present patent, it is disclosed a protocol that allows to produce NK cells in an approximately concentration of 1×10⁸ NK cells per ml, with more than 95% of cells being CD56+, and 60%-90% of cells being CD16+. These NK cells are called eNK. The Authors have developed a new method to obtain and arm EVs from expanded NK cells, and such method is described in the present patent as well. It is believed that the unique and distinctive characteristics of this method allow us to obtain EVs with unique and distinctive characteristics. This is the case for surface protein CD16, which expression has been reported in the prior art as not detected in EVs (Federici C, et al. Front Immunol. 2020; Lugini L, et al. J Immunol. 2012). Since eNK-derived EV population obtained through the disclosed method expresses proteins such as CD56 and CD16, it has been assumed that the prevalence of those proteins in the EV population mirror the eNK cell population. (Villalba M, et al. Research Square 2024). The in vitro method of obtaining the claimed EVs also discloses the steps for complexing EV to a Fc-modified recombinant polypeptide that displays high affinity for the membrane protein CD16. Although the quantification of the CD16 on EVs was not possible due to technical challenges, the fact that the Fc modified Ab is able to be effectively loaded onto EVs, tells us that surface CD16 concentration is sufficient. The binding of the CD16 membrane protein with the specific Ab through the Ab Fc modified domain results in what we termed "armed EVs".

Although cytotoxic granules are also considered exosomes, the present invention makes clear that cytotoxic granules are not present in the EVs described herein. Upon contacting with its target, the NK cell releases the cytotoxic granules in the intercellular space of the immune synapse, which in turn ensures that the lytic particles do not spread through surrounding areas. The cytotoxic granules will then fuse with the plasma membrane to release their content and kill the target cell. Since the production method of NK-EV is performed in the absence of target cells, the resulting NK-EVs do not contain cytotoxic granules. This is further supported by the absence of lytic granule degranulation (CD107a+ cells), which is absent in the eNK production conditions in which the NK-EVs are produced (Coënon J, et al. Immunother Cancer. 2024).

The present invention is firstly directed to an EV derived from a CD16+ cell, complexed to at least one recombinant polypeptide capable of binding to a FcγRIII/CD16 surface protein, wherein the recombinant polypeptide is non-covalently bound to the FcyRIII/CD16 surface protein located on the vesicle, and wherein said recombinant polypeptide comprises:
(i) a modified CH2 domain of a wild-type human IgG1 represented by SEQ ID NO 1; wherein the modified CH2 domain comprises amino acid substitutions S239D and I332E with respect to the CH2 domain of a wild-type human IgG1; bound to
(ii) a ligand binding domain, wherein the ligand binding domain comprises a sequence capable of binding to a target ligand.

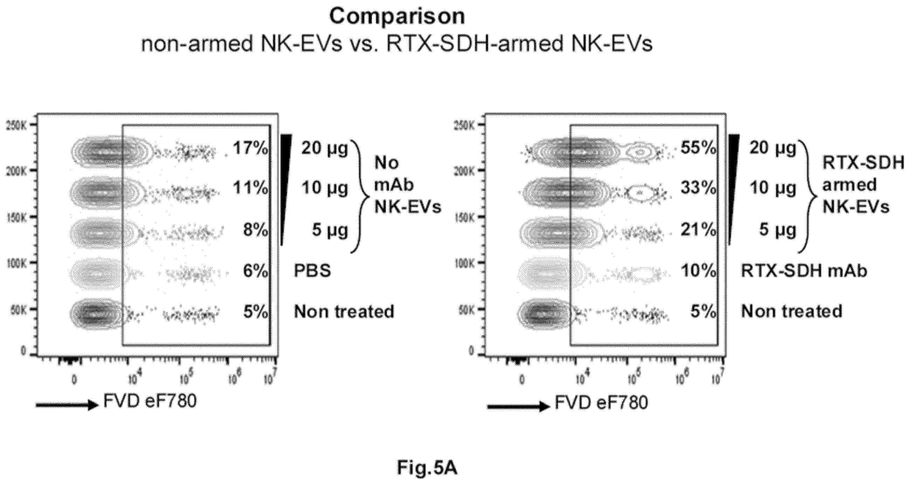

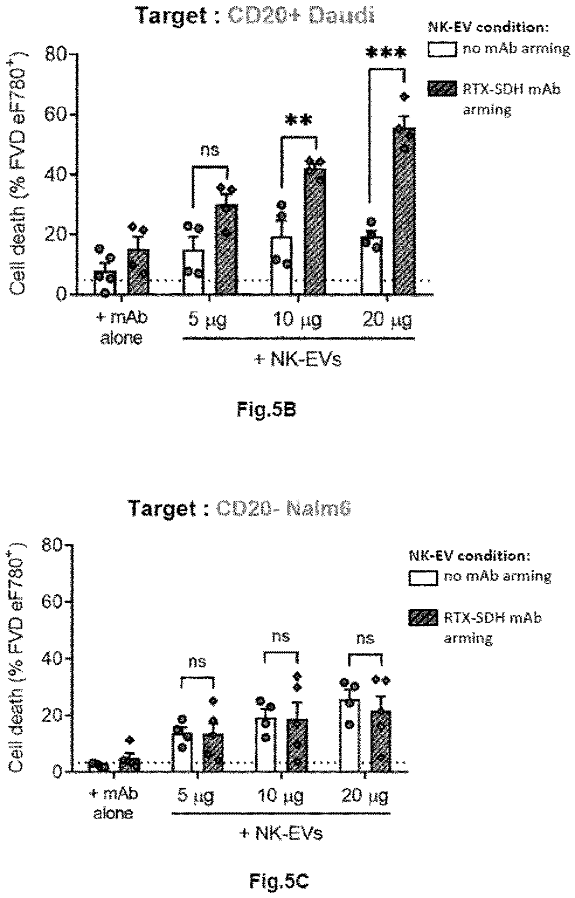

The success of the present invention relies on the stable manner in which the CD16+ EV is armed with a recombinant polypeptide comprising a modified Fc region. Giving selectivity to EVs by arming them with Fc-modified mAbs is a way to facilitate a greater and more efficient EV binding to target cells, overcoming the existing problem of arming EVs with non-modified Abs, which results in non-functional EV in terms of target activity and specificity towards target cells. Figures 5-6 of the present application are crucial to understand that SDH-armed EVs have significantly better efficacy compared to non-armed EVs or Wt-armed EVs.

Figure 5 represents the efficacy of SDH-RTX-armed EVs compared to non-armed EVs. If the Ab is really making the EVs recognize and engage with the target cells, so they can exert their cytotoxic activity on them, it would be expected that the efficacy of SDH-RTX-armed EVs to be better than non-armed EVs. Figure 5 shows the cytotoxic function of armed EVs on suspension cells compared to non-armed EVs. This experiment measures the percentage of CD20+ Daudi cell death after treatment with SDH-RTX-armed EVs vs. non-armed EVs. 20 µg of SDH-RTX-armed EVs were able to produce 55% of Daudi cell death compared to 17% of cell death produced by the same amount of non-armed EVs (Fig. 5A and Fig. 5B), concluding that the cytotoxic activity of armed EVs is more efficacious compared to non-armed EVs due to the existing significant difference between the two treatments. Fig 5C measures the percentage of CD20- Nalm6 cell death after treatment with SDH-RTX-armed EVs vs. non-armed EVs. This time around, there was no significant difference between NK-EVs with or without mAbs which produced the same amount of Nalm6 cell death. NK-EVs carrying RTX-SDH induced higher cell death on CD20+ Daudi cells, in comparison to control NK-EVs with no mAbs, in a dose-dependent manner. These results support that the antigen specificity of the mAb loaded on NK-EVs is the determinant factor that favors mAb armed NK-EVs over the control non-armed EVs. This enhanced specificity is also determined by the antigen expression on the surface of the target cells. These results suggest that parental NK cell arming with mAbs confers enhanced cytotoxicity to NK-derived EVs exclusively against targets expressing the antigen recognized by the mAb.

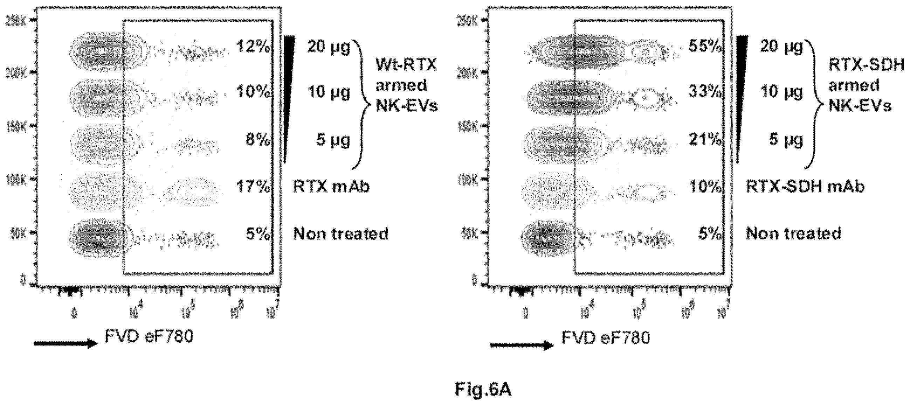

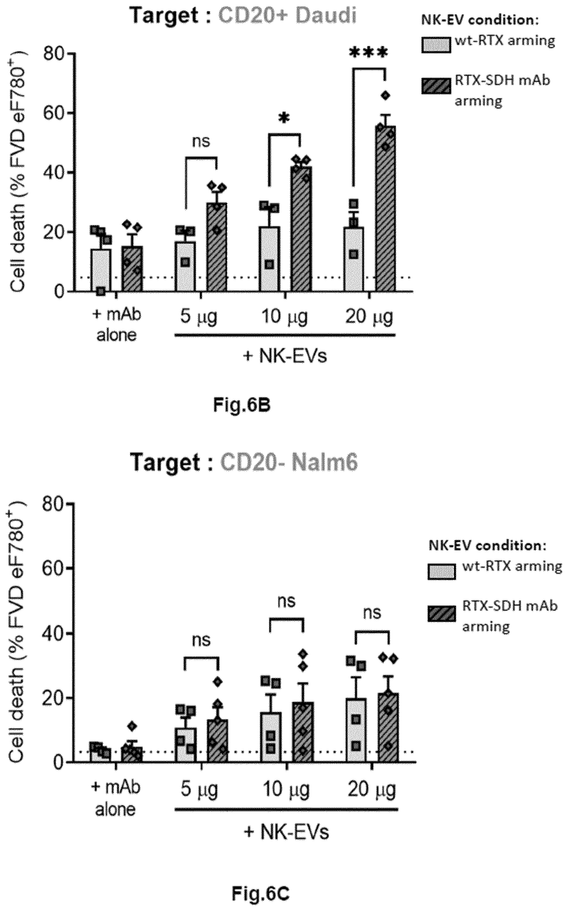

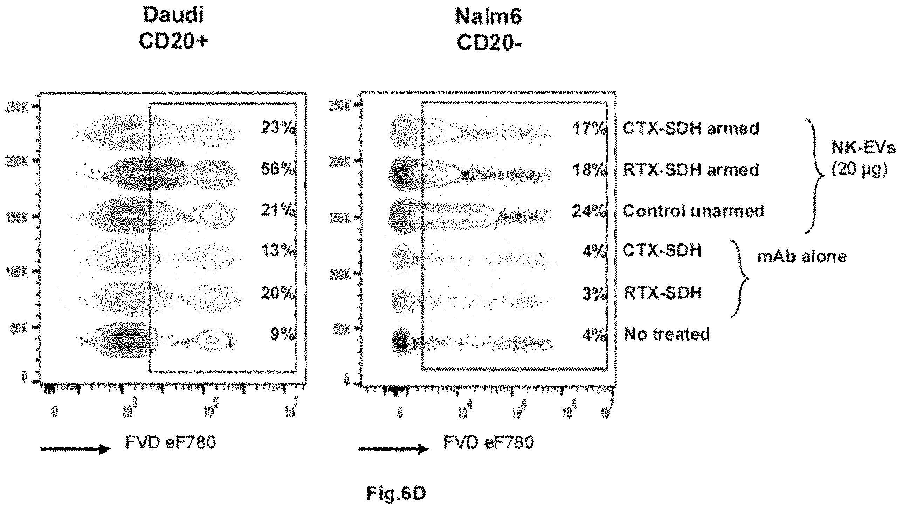

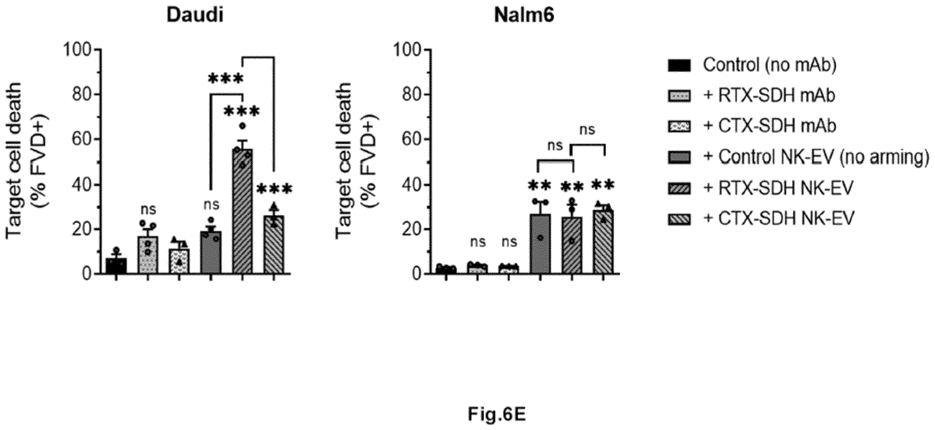

Figure 6 represents the efficacy of SDH-RTX-armed EVs compared to Wt-RTX-armed EVs showing the cytotoxic function of SDH-RTX-armed EVs on suspension cells compared to wt-RTX-armed EVs. This experiment measures the percentage of CD20+ Daudi cell death after treatment with SDH-RTX-armed EVs vs. wt-RTX-armed EVs. Prior experiments show that arming the EVs with an mAb enhances the cytotoxic activity of said EV and also improves EV specificity towards those cells that express the antigen recognized by the mAb. Figure 6 shows the experiments that the authors performed to show efficacy differences when modified mAbs were loaded on EVs compared to wt-mAbs. Unexpectedly, the treatment with NK-EVs carrying RTX-SDH induced higher cell death on CD20+ Daudi cells, in comparison to control wt-RTX-armed NK-EVs, in a dose-dependent manner. 20 µg of SDH-RTX-armed EVs were able to produce 55% of Daudi cell death compared to 12% of cell death produced by the same amount of wt-armed EVs (Fig. 6A and Fig. 6B), concluding that the cytotoxic activity of the SDH-armed EVs treatment is more efficacious compared to wt-RTX-armed EVs due to the existing significant difference between the two treatments. Fig 6C measures the percentage of CD20- Nalm6 cell death after treatment with SDH-RTX-armed EVs vs. wt-RTX-armed EVs. This time around, there was no significant difference between NK-EVs with or without mAbs which produced the same amount of Nalm6 cell death. The results in Fig. 6A and 6B show that the treatment is more specific and efficacious when the EV is armed with the SDH-modified antibody. The percentage of Daudi cell death after treatment is up to 7 times greater when EVs are armed with SDH-RTX compared to Wt-RTX. These results support that SDH modified mAb is the determinant factor that favors the better cytotoxic function observed on SDH-mAb-armed EVs compared to wt-mAb-armed EVs, and that we can enhance EV specificity not only by arming the EV with a specific antibody, but also by modifying the antibody domain that is bound to CD16.

Despite the results having been broken into 2 figures, the experiments from Figure 5A and Figure 6A have been done together under the same conditions and at same time points. If we take into account the results from Figure 5A and 6A as a whole, and we set side by side the efficacy of non-armed EVs and wt-RTX-armed EVs, results show that the percentage of CD20+ Daudi cell death after both treatments are very similar. 20 µg of non-armed EVs were able to produce 17% of Daudi cell death compared to 12% of cell death produced by the same amount of wt-armed EVs (Fig. 5A and Fig. 6A), concluding that wt-arming does not increase EV specificity or the cytotoxic activity compared to non-armed EV. On the other hand, SDH-RTX-armed EV treatment produces a greater percentage of CD20+ Daudi cell death, which supports that this treatment presents a better specificity and has significantly greater cytotoxicity compared to both non-armed EV and wt-RTX-armed EVs treatments. All these results together suggest that wt-arming does not confer any advantage in cytotoxic activity nor specificity of EVs compared to the non-armed strategy. It is in fact, SDH-arming strategy the one that improves not only specificity but also the cytotoxic activity compared to both non-armed and wt-arming strategies, getting those unexpected Daudi cell death rates that are up to up to 7 times greater when EVs are armed with SDH-RTX compared to Wt-RTX.

The present invention utilizes a recombinant polypeptide configured to bind to a CD16+ EV. It has been shown that the recombinant polypeptide attachment to the EVs is stable, and even suitable in therapy, when the recombinant polypeptide is bound non-covalently. Accordingly, "binding" or "attachment" of the recited recombinant polypeptide to a CD16+ EV is preferably considered as "non-covalent".

In the context of the present invention, "CD16+ cells" means a cell or population of cells that is "positive" for FcγRIII/CD16 and refers to the detectable presence on or in the cell of FcyRIII/CD16. CD16+ cell from any origin, preferably mammalian and more preferably human. "CD16+ cell" also refers to unmodified cells naturally expressing FcyRIII/CD16 or transiently modified cells to allow expression or overexpression of FcγRIII/CD16 or stably transformed cells to express or overexpress the FcγRIII/CD16. The transient or stable modifications refer to well-known modifications from the scientific knowledge such as but not limited to expose cells to chemical or biological reagent(s) or to genetically modified cells by transfecting nucleotides agents. For instance, the term "CD16+ cells" encompasses natural killer (NK) cells, neutrophils, monocytes, dendritic cells and macrophages.

The CD16+ cell surface marker is also referred herein as the FcγRIII surface receptor.

In the context of the present invention, a "ligand of Fc region of an IgG" means a molecule, preferably a polypeptide, suitable for binding the Fc region of an IgG-type, particularly of an IgG1-type, antibody to form a non-covalent complex. In a non-limiting manner, such Fc ligands include the polypeptides FcyRs, FcRn, Clq, C3, mannan-binding lectin, mannose receptor, staphylococcal protein A, streptococcal protein G, and viral origin FcγR. Fc ligands particularly include the Fc receptor homologs (FcRH), (Davis RS, et al. Immunological Revi. 2002). "Fcy receptor" or "Fc receptor" or "FcyR", or "FcgammaR", mean any member of the protein family encoded by an FcγR gene and suitable for binding the Fc region of an IgG-type, and more particularly of an IgG1-type, antibody. In humans, this family includes, without limitation, FcγRI (CD64), which includes FcγRIa, FcγRIb, and FcγRIc isoforms; FcγRII (CD32), including FcyRlla (including the H131 and R131 allotypes), FcγRIIb (including FcγRIIb- 1 and FcγRIIb-2), and FcγRIIc isoforms; and FcγRIII (CD16), including FcγRIIIa (including VI 58 and FI 58 allotypes) and FcγRIIIb (including FcγRIIb-NA 1 and FcγRIIb-NA2 allotypes) isoforms. An FcγR can be from any kind of organism, including specifically humans, mice, rats and monkeys.

Most particularly the FcγR receptors suitable for recognizing a modified Fc region according to the invention are the FcγRIII (CD16) receptors and isoforms thereof.

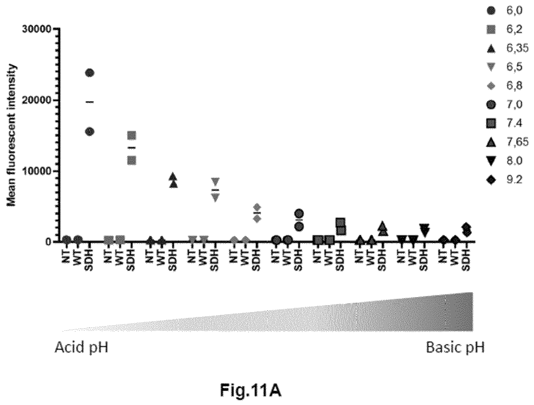

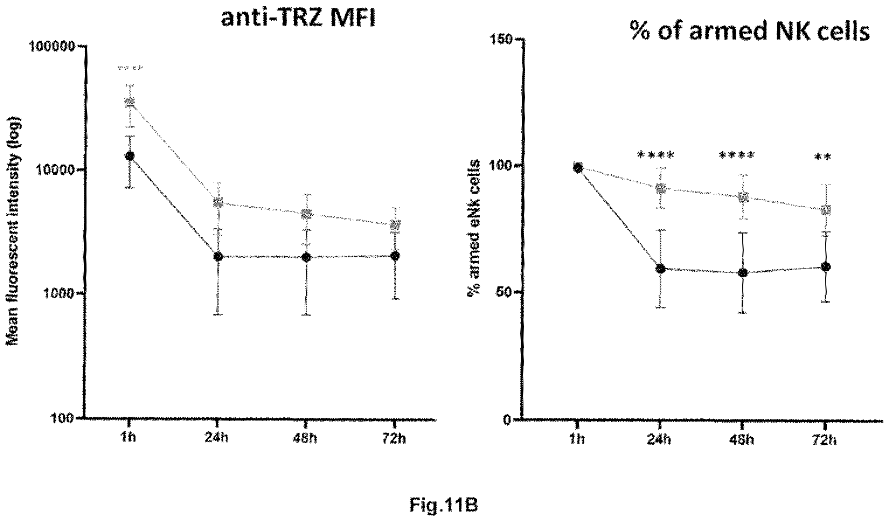

The human IgG1 Fc receptor FcγRIII (CD16) consists of two isoforms (CD16a/FcγRIIIla and CD16b/FcγRIIIb) that are encoded by two highly homologous genes. One of those isoforms, the integral membrane protein CD16a is predominantly expressed on NK cells and monocytes, and is of particular interest because it is the primary receptor responsible for antibody-dependent cell-mediated cytotoxicity and, thus, tumor clearance. Proteins located at the cell surface not only mediate intercellular interactions but also reveal fundamental cellular of its lineage, and are often critical to cell function. One good example is the protein FcγRllla/CD16a, which is expressed on NK cells and binds immunoglobulin G (IgG), triggering a protective immune response. IgG1 from serum is likely the predominant ligand for CD16a under normal conditions. It must be taken into account that every individual protein at the cell surface is potentially unique because of the compositional variability of post-translational modifications (PTMs) at multiple sites that are not directly encoded in the genome. Further, composition of the PTMs impacts protein function and possibly immune cell response including CD16a on NK cells. Little is known about CD16a processing, though composition impacted the affinity for IgG1 Fc in vitro. Presenting minimally remodeled N-glycans at N162 increased affinity by up to 50-fold for IgG1 Fc. It has been shown that N45 glycosylation also contributes to affinity by stabilizing CD16a structure; However, roles for the three remaining N-glycans in the CD16 structure remain undefined. Thus, there is a possibility that CD16a PTM composition impacts antibody binding affinity in vivo (Patel, et al. Mol Cell Proteomics. 2019). To date, it has not been yet demonstrated that CD16a has the same glycosylation pattern on both EV-NK and NK cell membrane. However, given that CD16a on EV-NKs and NK cells exhibit a very high affinity for IgG1 Fc, and that since the PTM composition of CD16a affects the binding affinity for antibodies in vivo, it makes sense to think that the glycosylation patterns of CD16a on EV-NKs and NK cells are so similar that they result in no affinity change for Fc. In addition, the CD16a expressed on NK-EVs has passed through endosomes where pH values can be as low as 4.0-4.5. It is demonstrated that acidic pH is able of not only changing the protein glycosylation pattern but even the tridimensional structure of the protein (Wang, et al. Eur J Pharm Sci 2008; Rayner. Exp Physiol. 1991), which in turn theoretically change the binding affinity between CD16 and the Fc protein domain. However, armed NK cells result in perfectly armed NK-EVs when obtained by the NK cell-derived extracellular vesicle isolation method described herein. So far, For the particular case of the SDHmAb-CD16a interaction, it is unknown the impact of the pH on the CD16/SDHmAb structure since there is no available literature data on how CD16-SDHmAB binding behaves in acidic PH. Authors have new data showing herein (Figure 11) that the SDHmAbs-CD16a interaction behaves stable in acidic Ph, and they show unexpected results on how acidic PH increases the stability of the SDHmAb-CD16a interaction compared to the wt-mAb-CD16 interaction. The Example 6 supports the advantage of modified antibodies when it comes to mAb-CD16a affinity. In the context of the present invention, "CD16+ EVs complexed to at least one recombinant polypeptide", which are also named "Armed CD16+ EVs", and they both mean a compound of construct (C) such as: [CD16+ EV] - [Recombinant polypeptide of formula (I)]. Therefore, "armed CD16+ EV" or "CD16+ EVs complexed to at least one recombinant polypeptide" encompasses a compound of construct (D): [CD16+ cell] - [[Modified CH2 domain] - [Linker]x - [Ligand binding domain]]. The terms "Armed CD16+ EVs" and "CD16+ EVs complexed to at least one recombinant polypeptide" are used indistinctively in the present text due to their equivalent meaning.

In each of the constructs C or D, the recombinant polypeptide of formula (I) is not attached to the CD16+ EV by a covalent bond. On the contrary, in each of the constructs (C) or (D) the peptide of formula (I) is attached to the CD16+ EV in a non-covalent manner, particularly by an association of the receptor/receptor ligand type.

In a polypeptide of formula (I), comprising a "ligand binding domain" unit, said "ligand" can be of any type, as long as (i) said ligand binding domain comprised in the recombinant polypeptide of formula (I) is capable of binding said ligand.

In the context of the present invention, a "recombinant polypeptide" or "antibody" refer to a polypeptide or polypeptide complex that specifically recognizes and binds to one or more antigens. An antibody can be a whole antibody or any antigen binding fragment or single chain thereof. Thus, the two terms "recombinant polypeptide" and "antibody" include any protein or peptide comprising at least a portion of an immunoglobulin molecule which has biological activity for binding to an antigen. An example includes, but is not limited to, the complementarity determining region (CDR) of the heavy/light chain or its ligand binding portion, the heavy or light chain variable region, the heavy or light chain constant region, the framework (FR) region or any portion thereof, or at least a portion of binding protein. Both terms "recombinant polypeptide" and "antibody" also encompass a polypeptide or polypeptide complex that possess antigen binding ability upon activation. The term "antibody fragment" or " recombinant polypeptide fragment" as used herein are part of an antibody, such as F(ab') 2, F(ab) 2, Fab', Fab, Fv, single -chain variable fragment (scFv), and the like; for example, diabodies. Regardless of the structure, the antibody fragment binds to the same antigen recognized by the intact antibody.

The terms "recombinant polypeptide fragment" or "antibody fragment" also include any synthetic or genetically engineered protein, or polypeptide, that acts like an antibody by binding to a specific antigen to form a complex. An antibody, an antigen binding polypeptide, or a variant or derivative thereof according to the invention includes, but is not limited to, a polyclonal antibody, mAb, multispecific antibody, human antibody, humanized antibody, primatized antibody or chimeric antibody, single-chain antibody, epitope-binding fragment (e.g., Fab, Fab' and F(ab') 2, Fd, Fvs, single-chain Fvs (scFv)), single -chain antibody, disulfide-linked Fvs (sdFv), fragment comprising a VK or VH domain, fragment generated from a Fab expression library, and anti-id antibody (including, for example, the anti-id antibody and LIGHT antibody disclosed herein). The antibody molecule of the invention may be an immunoglobulin molecule of any type (e.g., IgG, IgE, IgM, IgD, IgA, and IgY), species (e.g., IgG1, IgG2, IgG3, IgG4, IgAl, and IgA2) or subtype.

In the context of the present invention, "modified CH2 domain" means a polypeptide sequence corresponding to a modified form of a reference CH2 domain, such as a reference CH2 domain of a human IgG. Therefore, a modified CH2 domain in the sense of the invention differs from a reference sequence of an antibody CH2 domain, particularly of a human IgG1 CH2 domain, by one or several amino acid modifications. Such a modified region can relate indiscriminately to a recombinant polypeptide, to a composition (for example, pharmaceutical composition) comprising said recombinant polypeptide. The IgG Fc region comprises two CH2 and two CH3 domains.

Herein, the expression "CH2 domain of a wild-type human IgG1" relates to a part of the Fc region of human lgG1s that can be illustrated by the amino acid sequence of SEQ ID NO. 1 or a fragment of this SEQ ID NO. 1.

According to some embodiments of the present object of the invention, the modified CH2 domain has at least one amino acid modification with respect to the reference sequence (particularly the reference sequence of human IgG1), which encompasses at least one, two, three, four, five, or more than five modifications with respect to said reference sequence.

According to a preferred embodiment, the modified CH2 domain has an affinity with respect to the FcγRIII (CD16) surface protein expressed in CD16+ EVs selected from the group consisting of: modulated affinity, increased affinity and decreased affinity.

For all the positions discussed in the context of the present invention, and relative to modifications within the CH2 Domain, such as those defined in sequences SEQ ID NO. 2 to 7, with respect to SEQ ID NO. 1, and unless otherwise indicated, reference will be made to EU numbering by reference to Edelman (Edelman, et al. Proc Natl Acad Sci. 1969). Particularly, said modification can be an addition, a deletion or a substitution. A substitution can include specifically any natural or non-natural amino acid.

In the context of the present invention, "modification of an amino acid" means a substitution, an insertion and/or a deletion of an amino acid in a polypeptide sequence. "Amino acid substitution" or "substitution" means the replacement of an amino acid in a particular position in a wild-type polypeptide sequence with another amino acid. For example, the S239D substitution refers to a variant polypeptide, in this case an Fc variant, wherein serine in position 239 is replaced with aspartic acid. "Insertion of an amino acid" or "insertion" in the sense of the present document, the addition of an amino acid to a specific location in a parental polypeptide sequence. For example, the G > 235-236 insert designates an insertion of glycine between positions 235 and 236. "Deletion of an amino acid" or "deletion" herein means the elimination of an amino acid at a level in a parental polypeptide sequence. For example, G236 designates the deletion of glycine in position 236.

"Position" herein means a location in a protein or a polypeptide sequence. The positions can be numbered sequentially or according to an established format, for example the EU index such as in Kabat. For any position approached in the present invention, the numbering is according to EU numbering relating to the numbering of the EU antibody (Edelman, et al. Proc Natl Acad Sci. 1969). Correspondence with the Kabat numbering can be established by means of the correspondence table; IMGT unique numbering for C- DOMAIN.

In the context of the present invention, "ligand binding domain from an antibody" refers to a sequence capable of binding to a target ligand, such as a target substance, a target compound, a target molecule or even a target cell, (i) for example a ligand binding domain which is capable of binding to a specific antigen of a target cell, or a sequence capable of binding to a natural or tumor antigen, such as the antigen binding domain of an antibody, which is capable of binding to said target or (ii) for example a ligand binding domain which is capable of binding to a cell receptor or binding to the antigen binding domain of an antibody, in which case the ligand binding domain comprises, or consists of, an antigen recognized by the antigen binding domain of said antibodies.

The term "antigen binding fragment" or 'antigen binding domain" or 'ligand binding domain from an antibody", as used herein, refers in particular to one or more fragments of an intact antibody that retain the ability to specifically binds to a given antigen/ligand. Antigen binding functions of an antibody can be performed by fragments of an intact antibody. Examples of binding fragments encompassed within the term antigen binding fragment of an antibody include a Fab fragment, a monovalent fragment consisting of the VL, VH, CL and CHI domains; a Fab' fragment, a monovalent fragment consisting of the VL, VH, CL, CHI domains and hinge region; a L(ab')2 fragment, a bivalent fragment comprising two Lab' fragments linked by a disulfide bridge at the hinge region; an Ld fragment consisting of VH domains of a single arm of an antibody; a single domain antibody (sdAb) fragment (Ward et al. Nature. 1989), which consists of a VH domain or a VL domain; and an isolated complementary determining region (CDR). Furthermore, although the two domains of the Lv fragment, VL and VH, are coded for by separate genes, they can be joined, using recombinant methods, by an artificial peptide linker that enables them to be made as a single protein chain in which the VL and VH regions pair to form monovalent molecules (Bird, et al. Science. 1988; Huston et al. Proc. Natl. Acad. Sci. 1988). "dsFv" is a VH::VL heterodimer stabilized by a disulfide bond. Divalent and multivalent antibody fragments can form either spontaneously by association of monovalent scFvs, or can be generated by coupling monovalent scFvs by a peptide linker, such as divalent sc(Fv)2. Such single chain antibodies include one or more antigen binding portions or fragments of an antibody. These antibody fragments are obtained using conventional techniques known to those skilled in the art, and the fragments are screened for utility in the same manner as are intact antibodies. A unibody is another type of antibody fragment lacking the hinge region of IgG4 antibodies. The deletion of the hinge region results in a molecule that is essentially half the size of traditional IgG4 antibodies and has a univalent binding region rather than the bivalent binding region of IgG4 antibodies. Antigen binding fragments can be incorporated into single domain antibodies, SMIP, maxibodies, minibodies, intrabodies, diabodies, triabodies and tetrabodies (Hollinger and Hudson. Nat Biotechnol. 2005). The term "diabodies" "tribodies" or "tetrabodies" refers to small antibody fragments with multivalent antigen-binding sites (2, 3 or four), which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Antigen binding fragments can be incorporated into single chain molecules comprising a pair of tandem Fv segments (VH-CH1-VH-CH1) Which, together with complementary light chain polypeptides, form a pair of antigen binding regions (Zapata et al. Protein Eng. 1995; U.S. Pat. No. 5,641,870).

Preferably, the ligand binding domain comprised therein is capable of binding to a ligand defined by, but not limited to, the proteins, sub-units, domains, motifs and/or epitopes belonging to the following list of targets: 17-IA, 4-1BB, 4Dc, 6-keto-PGFIa, 8-iso-PGF2a, 8-oxo-dG, A1 Adenosine Receptor, A33, ACE, ACE-2, Activin, Activin A, Activin AB, Activin B, Activin C, Activin RIA, Activin RIA AFK-2, Activin RIB AFK-4, Activin RIIA, Activin RUB, ADAM, ADAM 10, ADAM 12, AD AMI 5, ADAM 17/T ACE, ADAM8, ADAM9, ADAMTS, ADAMTS4, ADAMTS5, Addressins, aFGF, AFCAM, AFK, AFK-1, AFK-7, alpha- 1 -antitrypsin, alpha- V/beta-1 antagonist, ANG, Ang, APAF- 1, APE, APJ, APP, APRIF, AR, ARC, ART, Artemin, anti-id, ASPARTIC, Atrial natriuretic factor, av/b3 integrin, Axl, b2M, B7-1, B7-2, B7-H, B-lymphocyte Stimulator (BlyS), BACE, BACE-1, Bad, BAFF, BAFF-R, Bag-1, BAK, Bax, BCA-1, BCAM, Bel, BCMA, BDNF, b-ECGF, bFGF, BID, Bik, BIM, BLC, BL-CAM, BLK, BMP, BMP-2 BMP-2a, BMP-3 Osteogenin, BMP-4 BMP- 2b, BMP-5, BMP-6 Vgr-1, BMP-7 (OP-1), BMP-8 (BMP-8a, OP-2), BMPR, BMPR-IA (ALK-3), BMPR-IB (ALK-6), BRK-2, RPK-1, BMPR-II (BRK-3), BMPs, b-NGF, BOK, Bombesin, Bone- derived neurotrophic factor, BPDE, BPDE-ADN, BTC, complement factor 3 (C3), C3a, C4, C5, C5a, CIO, CA125, CAD-8, Calcitonin, cAMP, Carcinoembryonic antigen (CEA), carcinoma-associated antigen, Cathepsin A, Cathepsin B, Cathepsin C/DPPI, Cathepsin D, Cathepsin E, Cathepsin H, Cathepsin L, Cathepsin O, Cathepsin S, Cathepsin V, Cathepsin X/Z/P, CBL, CCI, CCK2, CCL, CCL1, CCL11, CCL12, CCL13, CCL 14, CCL15, CCL 16, CCL17, CCL18, CCL19, CCL2, CCL20, CCL21, CCL22, CCL23, CCL24, CCL25, CCL26, CCL27, CCL28, CCL3, CCL4, CCL5, CCL6, CCL7, CCL8, CCL9/10, CCR, CCR1, CCR10, CCR10, CCR2, CCR3, CCR4, CCR5, CCR6, CCR7, CCR8, CCR9, CD1, CD2, CD3, CD3E, CD4, CD5, CD6, CD7, CD8, CD10, CDIIa, CDIIb, CDIIc, CD13, CD14, CD15, CD16, CD18, CD19, CD20, CD21, CD22, CD23, CD25, CD27L, CD28, CD29, CD30, CD30L, CD32, CD33 (p67 proteins), CD34, CD38, CD40, CD40L, CD44, CD45, CD46, CD49a, CD52, CD54, CD55, CD56, CD61, CD64, CD66e, CD74, CD80 (B7-1), CD89, CD95, CD123, CD137, CD138, CD140a, CD146, CD147, CD148, CD152, CD164, CEACAM5, CFTR, cGMP, CINC, Clostridium botulinum toxin, Clostridium perfringens toxin, CKb8-I, CLC, CMV, CMVUL, CNTF, CNTN-1, COX, C-Ret, CRG-2, CT-1, CTACK, CTGF, CTLA-4, CX3CL1, CX3CR1, CXCL, CXCL1, CXCL2, CXCL3, CXCL4, CXCL5, CXCL6, CXCL7, CXCL8, CXCL9, CXCL10, CXCL11, CXCL 12, CXCL 13, CXCL 14, CXCL15, CXCL 16, CXCR, CXCR1, CXCR2, CXCR3, CXCR4, CXCR5, CXCR6, cytokeratin tumor-associated antigen, DAN, DCC, DcR3, DC-SIGN, Decay accelerating factor, des(I-3)-IGF-I (brain IGF-1), Dhh, digoxin, DNAM-1, DNase, Dpp, DPPIV/CD26, Dtk, ECAD, EDA, EDA-A1, EDA-A2, EDAR, EGF, EGFR (ErbB-1), EM A, EMMPRIN, ENA, endothelin receptor, Enkephalinase, eNOS, Eot, eotaxinl, EpCAM, Ephrin B2/ EphB4, EPO, ERCC, E-selectin, AND-1, Factor Ila, Factor VII, Factor VIIIc, Factor IX, fibroblast activation protein (FAP), Fas, FcRI, FEN-1, Ferritin, FGF, FGF- 19, FGF-2, FGF3, FGF-8, FGFR, FGFR-3, Fibrin, FL, FLIP, Flt-3, Flt-4, Follicle stimulating hormone, Fractalkine, FZD1, FZD2, FZD3, FZD4, FZD5, FZD6, FZD7, FZD8, FZD9, FZD10, G250, Gas 6, GCP-2, GCSF, GD2, GD3, GDF, GDF-1, GDF-3 (Vgr-2), GDF-5 (BMP-14, CDMP-1), GDF-6 (BMP-13, CDMP-2), GDF-7 (BMP-12, CDMP-3), GDF- 8 (Myostatin), GDF-9, GDF-15 (MIC-1), GDNF, GDNF, GFAP, GFRa-1, GFR-alphal, GFR-alpha2, GFR-alpha3, GITR, Glucagon, Glut 4, glycoprotein TTb/TTT a (GP Ilb/Illa), GM-CSF, gpl30, gp72, GRO, Growth hormone releasing factor, Hapten (NP-cap or NIP-cap), HB-EGF, HCC, HCMV gB envelope glycoprotein, HCMV gH envelope glycoprotein, HCMV UL, Hemopoietic growth factor (HGF), Hep B gpl20, heparanase, Her2, Her2/neu (ErbB-2), Her3 (ErbB-3), Her4 (ErbB-4), herpes simplex virus (HSV) gB glycoprotein, HSV gD glycoprotein, HGFA, High molecular weight melanoma-associated antigen (HMWMAA), HIV gpl20, HIV IIIB gp 120 V3 loop, HLA, HLA-DR, HM1.24, HMFG PEM, HRG, Hrk, human cardiac myosin, human cytomegalovirus (HCMV), human growth hormone (HGH), HVEM, 1-309, IAP, ICAM, ICAM-1, ICAM-3, ICE, ICOS, IFNg, Ig, IgA receptor, IgE, IGF, IGF binding proteins, IGF-1R, IGFBP, IGF-I, IGF-II, IL, IL-1, IL-1R, IL-2, IL-2R, IL-4, IL-4R, IL-5, IL- 5R, IL-6, IL-6R, IL-8, IL-9, IL-10, IL-12, IL-13, IL-15, IL-18, IL-18R, IL-23, interferon (INF)- alpha, INFbeta, INF-gamma, Inhibin, iNOS, Insulin A-chain, Insulin B-chain, Insulin-like growth factor 1, integrin alpha2, integrin alpha3, integrin alpha4, integrin alpha4/betal, integrin alpha4/beta7, integrin alpha5 (alpha V), integrin alpha5/betal, integrin alpha5/beta3, integrin alpha6, integrin betal, integrin beta2, interferon gamma, IP-10, I-TAC, JE, Kallikrein 2, Kallikrein 5, Kallikrein 6, Kallikrein 11, Kallikrein 12, Kallikrein 14, Kallikrein 15, Kallikrein LI, Kallikrein L2, Kallikrein L3, Kallikrein L4, KC, KDR, Keratinocyte Growth Factor (KGF), laminin 5, LAMP, LAP, LAP (TGF-1), Latent TGF-1, Latent TGF-1 bpl, LBP, LDGF, LECT2, Lefty, Lewis-Y antigen, Lewis-Y related antigen, LFA-1, LFA-3, Lfo, LIF, LIGHT, lipoproteins, LlX, LKN, Lptn, L-Selectin, LT-a, LT-b, LTB4, LTBP-1, Lung surfactant, Luteinizing hormone, Lymphotoxin Beta Receptor, Mac-1, MAdCAM, MAG, MAP2, MARC, MCAM, MCAM, MCK-2, MCP, M-CSF, MDC, Mer, METALLOPROTEASES, MGDF receptor, MGMT, MHC (HLA-DR), MIF, MIG, MIP, MIP-1 -alpha, MK, MMAC1, MMP, MMP-1, MMP-10, MMP-11, MMP-12, MMP-13, MMP-14, MMP-15, MMP-2, MMP-24, MMP-3, MMP-7, MMP-8, MMP-9, MPIF, Mpo, MSK, MSP, mucin (Mud), MUC18, Muellerian-inhibitin substance, Mug, MuSK, NAIP, NAP, NCAD, N-Cadherin, NCA 90, NCAM, NCAM, Neprilysin, Neurotrophin-3,-4, or -6, Neurturin, Neuronal growth factor (NGF), NGFR, NGFbeta, nNOS, NO, NOS, Npn, NRG-3, NT, NTN, OB, OGGI, OPG, OPN, OSM, OX40L, OX40R, pl50, p95, PADPr, Parathyroid hormone, PARC, PARP, PBR, PBSF, PCAD, P- Cadherin, PCNA, PDGF, PDGF, PDK-1, PECAM, PEM, PF4, PGE, PGF, PGI2, PGJ2, PIN, PLA2, placental alkaline phosphatase (PLAP), PIGF, PLP, PP14, Proinsulin, Prorelaxin, Protein C, PS, PSA, PSCA, prostate specific membrane antigen (PSMA), PTEN, PTHrp, Ptk, PTN, R51, RANK, RANKL, RANTES, RANTES, Relaxin A-chain, Relaxin B-chain, renin, respiratory syncytial virus (RSV) F, RSV Fgp, Ret, Rheumatoid factors, RLIP76, RPA2, RSK, S100, SCF/KL, SDF-1, SERINE, Serum albumin, sFRP-3, Shh, SIGIRR, SK-1, SLAM, SLPI, SMAC, SMDF, SMOH, SOD, SPARC, Stat, STEAP, STEAP-II, TACE, TACI, TAG-72 (tumor-associated glycoprotein-72), TARC, TCA-3, T-cell receptors (e.g., T-cell receptor alpha/beta), TdT, TECK, TEM1, TEM5, TEM7, TEM8, TERT, testicular PLAP-like alkaline phosphatase, TfR, TGF, TGF-alpha, TGF-beta, TGF-beta Pan Specific, TGF-beta RI (ALK-5), TGF-beta RII, TGF-beta Rllb, TGF-beta RIII, TGF- beta 1, TGF-beta2, TGF-beta3, TGF-beta4, TGF-beta5, Thrombin, Thymus Ck-1, Thyroid stimulating hormone, Tie, TIMP, TIQ, Tissue Factor, TMEFF2, Tmpo, TMPRSS2, TNF, TNF-alpha, TNF-alpha beta, TNF-beta2, TNFc, TNF-RI, TNF-RII, TNFRSF10A (TRAIL RI Apo-2, DR4), TNFRSF10B (TRAIL R2 DR5, KILLER, TRICK-2A, TRICK-B), TNFRSF10C (TRAIL R3 DcRI, LIT, TRID), TNFRSF10D (TRAIL R4 DcR2, TRUNDD), TNFRSF11A (RANK ODF R, TRANCE R), TNFRSF11B (OPG OCIF, TR1), TNFRSF12 (TWEAK R FN14), TNFRSF13B (TACI), TNFRSF13C (BAFF R), TNFRSF14 (HVEM ATAR, HveA, LIGHT R, TR2), TNFRSF16 (NGFR p75NTR), TNFRSF17 (BCMA), TNFRSF18 (GITR AITR), TNFRSF19 (TROY TAJ, TRADE), TNFRSF19L (RELT), TNFRSF1A (TNF RI CD120a, p55-60), TNFRSF1B (TNF RII CD120b, p75- 80), TNFRSF26 (TNFRH3), TNFRSF3 (LTbR TNF RIII, TNFC R), TNFRSF4 (0X40 ACT35, TXGP1 R), TNFRSF5 (CD40 p50), TNFRSF6 (Fas Apo-1, APT1, CD95), TNFRSF6B (DcR3 M68, TR6), TNFRSF7 (CD27), TNFRSF8 (CD30), TNFRSF9 (4-1 BB CD137, ILA), TNFRSF21 (DR6), TNFRSF22 (DcTRAIL R2 TNFRH2), TNFRST23 (DcTRAIL RI TNFRH1), TNFRSF25 (DR3 Apod, LARD, TR-3, TRAMP, WSL-1), TNFSF10 (Ligand Apo-2 TRAIL, TL2), TNFSF11 (Ligand TRANCE/RANK ODF, OPG Ligand), TNFSF12 (TWEAK Apo-3 Ligand, DR3 Ligand), TNFSF13 (APRIL TALL2), TNFSF13B (BAFF BLYS, TALL1, THANK, TNFSF20), TNFSF14 (LIGAND HVEM LIGHT, LTg), TNFSF15 (TL1A/VEGI), TNFSF18 (GITR Ligand AITR Ligand, TL6), TNFSF1A (TNF-a Conectine, DIF, TNFSF2), TNFSF1B (TNF-b LTa, TNFSF1), TNFSF3 (LTb TNFC, p33), TNFSF4 (0X40 Ligand gp34, TXGP1), TNFSF5 (CD40 Ligand CD154, gp39, HIGM1, IMD3, TRAP), TNFSF6 (Fas Ligand Apo-1 Ligand, APT1 Ligand), TNFSF7 (CD27 Ligand CD70), TNFSF8 (CD30 Ligand CD153), TNFSF9 (4-1 BB Ligand CD137 Ligand), TP-1, t- PA, Tpo, TRAIL, TRAIL R, TRAIL-R1, TRAIL-R2, TRANCE, transfert receptor, TRF, Trk, TROP- 2, TSG, TSLP, CA 125 tumor-associated antigen, tumor-associated antigen expressing Lewis Y- related carbohydrate, TWEAK, TXB2, Ung, uPAR, uPAR-1, Urokinase, VCAM, VCAM-1, VECAD, VE-Cadherin, VEcadherin- 2, VEFGR-1 (flt-1), VEGF, VEGFR, VEGFR-3 (flt-4), VEGI, VIM, Viral antigens, VLA, VLA-1, VLA-4, VNR integrin, von Willebrands factor, WIF-1, WNT1, WNT2, WNT2B/13, WNT3, WNT3A, WNT4, WNT5A, WNT5B, WNT6, WNT7A, WNT7B, WNT8A, WNT8B, WNT9A, WNT9A, WNT9B, WNT10A, WNT10B, WNT11, WNT16, XCL1, XCL2, XCR1, XCR1, XEDAR, XIAP, XPD, and hormone and growth factor receptors, etc.

"IgG" herein means a polypeptide belonging to the class of antibodies which are essentially encoded by a recognized gamma immunoglobulin gene. In humans, this IgG comprises the IgG1, IgG2, IgG3 and IgG4 sub-classes or isotypes. In mice, the IgGs comprise IgG1, IgG2a, IgG2b, IgG3. "Isotype" herein means any immunoglobulin subclass defined by the chemical and antigenic characteristics of its constant regions. The known isotypes of human immunoglobulins are as follows: IgG1, IgG2, IgG3, IgG4, IgAI, IgA2, IgM, IgD and IgE.

"Target cell" or "target ligand" herein means a cell expressing a target antigen that can be recognized by an armed NK-EV according to the invention or a cell expressing an antibody capable of recognizing an antigen presented by an armed NK-EV according to the invention. For example, a target cell can be a cancer cell, a cell infected by a pathogenic agent, an autoreactive B-cell, an autoreactive T-cell.

"Wild-type or WT" means herein an amino acid sequence which is found in nature, including allele variations. A WT protein, a polypeptide, an antibody, an immunoglobulin, an IgG, etc., an amino acid sequence or a nucleotide sequence which has not been intentionally modified.

In a preferred embodiment, the modified CH2 domain comprises at least one additional amino acid substitution selected from the group consisting of H268F, S324T, G236A, A330L, and any combination thereof.

More preferably, the modified CH2 domain comprises an amino acid sequence having at least 95% sequence identity with a CH2 domain of a human IgG, for example, a CH2 domain of a wild- type human IgG1. The CH2 domain can contain other modifications (for example, reducing or eliminating the effector function).

It is a preferred embodiment a modified CH2 domain that comprises at least one amino acid substitution in a position selected from the group consisting of: 230, 233, 234, 235, 236, 239, 240, 243, 264, 266, 268, 272, 274, 275, 276, 278, 302, 318, 324, 325, 326, 327, 328, 329, 330, 331, 332 and 335, where the amino acid numbering in the Fc region is according to EU numbering. In certain preferred embodiments, said modified CH2 domain comprises at least one amino acid substitution selected from the group consisting of: P230E, P230Y, P230G, E233N, E233Q, E233K, E233R, E233S, E233T, E233H, E233A, E233V, E233L, E233I, E233F, E233M, E233Y, E233W, E233G, L234K, L234R, L234S, L234A, L234M, L234W, L234P, L234G, L234A, L235E, L235K, L235R, L235A, L235A, L235M, L235W, L235P, L235G, G236D, G236E, G236A, G236N, G236Q, G236K, G236R, G236S, G236T, G236H, G236A, G236V, G236L, G236I, G236F, G236M, G236Y, G236W, G236P, S239Q, S239K, S239R, S239V, S239L, S239I, S239M, S239D, S239W, S239P, S239G, F243E, V264D, V264E, V264N, V264Q, V264K, V264R, V264S, V264H, V264W, V264P, V264G, H268D, H268E, H268Q, H268K, H268R, H268T, H268V, H268L, H268I, H268F, H268M, H268W, H268P, H268G, E272D, E272R, E272T, E272H, E272V, E272L, E272F, E272M, E272W, E272P, E272G, K274D, K274N, K274S, K274H, K274V, K274I, K274F, K274M, K274W, K274P, K274G, F275L, N276D, N276T, N276H, N276V, N276I, N276F, N276M, N276W, N276P, N276G, 278D, Y278N, Y278Q, Y278R, Y278S, Y278H, Y278V, Y278L, Y278I, Y278M, Y278P, Y278G, E318Q, E318H, E318L, E318Y, S324H, S324F, S324M, S324W, S324P, S324G, S324T, N325K, N325R, N325S, N325F, N325M, N325Y, N325W, N325P, N325G, K326P, A327E, A327K, A327R, A327H, A327V, A327I, A327F, A327M, A327Y, A327W, A327P, L328D, L328Q, L328K, L328R, L328S, L328T, L328V, L328I, L328Y, L328W, L328P, L328G, P329D, P329E, P329N, P329Q, P329K, P329G, P329R, P329S, P329T, P329H, P329V, P329L, P329I, P329M, P329Y, P329W, P329G, A330E, A330L, A330N, A330T, A330P, A330G, P331D, P331Q, P331R, P331T, P331L, P331I, P331F, P331M, P331Y, P331W, I332K, I332R, I332S, I332E, I332V, I332L, I332F, I332M, I332W, I332P, I332G, E333L, E333F, E333M, E333P, K334P, T335N, T335S, T335H, T335V, T335L, T335I, T335F, T335M, T335W, T335P, T335G, where the amino acid numbering in the Fc region is according to EU numbering.

In a preferred embodiment, the modified Fc region and/or the CH2 domain of the modified Fc region comprise at least one amino acid substitution selected from the group consisting of S239D (SEQ ID NO. 2), I332E (SEQ ID NO. 3), H268F (SEQ ID NO. 4), S324T (SEQ ID NO. 5), G236A (SEQ ID NO. 6), A330L (SEQ ID NO. 7), and any combination thereof, according to EU numbering, with respect to the Fc WT region, that is, the CH 2 domain of a wild-type human IgG1 (for example, SEQ ID NO. 1).

In view of the above, the amino acid sequence of the modified Fc region and/or the CH 2 domain of the modified Fc region correspond to the amino acid sequence presented in any one of SEQ ID NO. 2 to 7

Preferably, the recombinant polypeptide according to the invention comprises a modified Fc region, wherein the CH2 domain contains or does not other modifications.

Also preferably, the recombinant polypeptide according to the invention comprises a modified Fc region, wherein the hinge region can be a hinge region of wild-type IgG1 with or without substitutions.

According to a particular, preferred embodiment, the modified CH2 domain comprises the amino acid substitutions S239D, I332E, H268F, and S324T, with respect to the CH2 domain of a wild-type human IgG1.

In another particular embodiment, the modified Fc region and/or the CH2 domain of the modified Fc region comprise amino acid substitutions S239D, H268F, S324T, and I332E (SEQ ID NO. 8), referred to as Fc SDH region, according to EU numbering, with respect to the Fc WT region, that is, the CH2 domain of a wild-type human IgG1 (for example, SEQ ID NO. 1).

Preferably, the FcγRIII (CD16) surface protein is a FcγRllla/CD16a surface protein or a FcγRIIIb/CD16b surface protein
The human IgG Fc receptor CD16 (FcγRIII) consists of two isoforms, CD16a/FcγRIIIa and CD16b/FcγRIIIb, that are encoded by two highly homologous genes. CD16a is an integral membrane protein predominantly expressed on NK cells and monocytes. CD16b is predominantly expressed with a glycosylphosphatidylinositol anchor on neutrophils, a subset of basophils and shows inducible expression on eosinophils. CD16a and CD16b share 97% sequence identity and differ by only four amino acid residues in the extracellular antibody-binding domains (Roberts and Barb. J Biol Chem. 2018; Wang, et al. Biochim Biophys Acta. 2013). In the context of the present invention, the "FcγRIIIa/CD16a surface protein" refers to the activating receptor CD16a, also known as FcγRIIIa, mainly expressed on the cell surface of immune cells. In the context of the present invention, the "FcγRIIIb/CD16b protein" refers to the glycosylphosphatidylinositol (GPI) anchored glycoprotein CD16b, also known as FcγRIIIb, expressed only by neutrophils and it primarily recognizes IgG-containing immune complexes.

Preferably, the EV is derived from a CD16+ cell that is allogeneic or autologous with respect to an individual in need thereof.

In the context of the present invention, "CD16+ cell" means a cell that expresses the FcγRIII (CD16) surface protein, preferably allogeneic or autologous with respect to an individual in need thereof. According to some embodiments the "CD16+ cell" is furthermore in combination with a recombinant polypeptide; comprising (i) a modified Fc region or a variant thereof, preferably comprising a modified CH2 domain, and (ii) a ligand binding domain. "Armed CD16+ cell" means a compound of construct (A) such as: [CD16+ cell] - [Recombinant polypeptide of formula (I)]. Therefore, "armed CD16+ cell" encompasses a compound of construct (B): [CD16+ cell] - [[Modified Fc region] - [Linker]x - [Ligand binding domain]]. According to some embodiments, the armed-EVs could be obtained directly from "Armed CD16+ cell."

In each of the constructs A or B, the recombinant polypeptide of formula (I) is not attached to the CD16+ cell by a covalent bond. On the contrary, in each of the constructs (A) or (B) the peptide of formula (I) is attached to the CD16+ cell in a non-covalent manner, particularly by an association of the receptor/receptor ligand type.

In a polypeptide of formula (I), comprising a "ligand binding domain" unit, said "ligand" can be of any type, as long as (i) said ligand binding domain comprised in the recombinant polypeptide of formula (I) is capable of binding said ligand.

In the context of the present invention, "armed NK cell" means a NK cell, preferably allogeneic or autologous with respect to an individual in need thereof, in combination with a recombinant polypeptide; comprising (i) a modified Fc region or a variant thereof, preferably comprising a modified CH2 domain, and (ii) a ligand binding domain. "Armed NK cell" means a compound of construct (A) such as: [NK cell] -

[Recombinant polypeptide of formula (I)]. Therefore, "armed NK cell" encompasses a compound of construct (B): [NK cell] - [[Modified Fc region] - [Linker]x - [Ligand binding domain]]. According to some embodiments, the armed- NK-EV could be obtained directly from "Armed NK cell."

In each of the constructs A or B, the recombinant polypeptide of formula (I) is not attached to the NK cell by a covalent bond. On the contrary, in each of the constructs (A) or (B) the peptide of formula (I) is attached to the NK cell in a non-covalent manner, particularly by an association of the receptor/receptor ligand type.

In a polypeptide of formula (I), comprising a "ligand binding domain" unit, said "ligand" can be of any type, as long as (i) said ligand binding domain comprised in the recombinant polypeptide of formula (I) is capable of binding said ligand.

In certain embodiments of a polypeptide of formula (I), the ligand binding domain can consist of a molecule recognized by a receptor, for example a molecule recognized by a receptor expressed by a target cell, or even a molecule recognized by the antigen binding domain of an antibody. In the last case, the ligand binding domain comprised in a recombinant polypeptide of formula (I), which is recognized by the antigen binding domain of an antibody, can also be referred to as "antigen".

In other embodiments of a polypeptide of formula (I), the ligand binding domain comprises an antigen binding domain of an antibody. In these other embodiments, the ligand is a molecule recognized by said antibody binding domain, such as a molecule expressed by a target cell, for example a target tumor antigen, a target cell marker protein, or even a target cell receptor.

In a particular embodiment, the CD16+ cells used in the invention to obtain EVs, are cells from a human other than the individual to be treated, referred to as allogeneic.

In another particular embodiment, the CD16+ cells used in the invention to obtain EVs are cells from the individual himself or herself to be treated, referred to as autologous.

Preferably, the modified CH2 domain is bound to the ligand binding domain through a linker.

In the context of the present invention, the linker comprises a polypeptide sequence having 1 to 100 amino acids.

In a preferred embodiment, the recombinant polypeptide comprises a human IgG1 Fc region comprising the modified CH2 domain.

Preferably, the recombinant polypeptide comprises (i) a modified Fc region, which improves the binding properties of the recombinant polypeptide with respect to the same polypeptide in wild-type form, and (ii) a ligand binding domain. For example, a polypeptide comprising such a modified Fc region has an increased stability and binding specificity with respect to an Fc receptor, more particularly with respect to the FcγRIII receptor (CD16). Furthermore, a recombinant polypeptide comprising such a modified Fc region allows stabilizing said polypeptide at the EV membrane inhibiting the internalization thereof. Therefore, the recombinant polypeptides of the present invention are optimized versions of the parent non-modified polypeptide, that is, the wild-type polypeptide. More precisely, the recombinant polypeptide comprises (i) an Fc region consisting of the constant heavy chain portion beyond the hinge portion comprising two CH2 and CH3 domains (CH2 domain and/or CH3 domain), allowing attachment to Fc receptors and (ii) a region capable of binding to a ligand, allowing recognition of target cells.

In the context of IgG antibodies, each of the IgG isotypes has a Y-shaped unit composed of four polypeptide chains two identical copies of a heavy chain (H) and two identical copies of a light chain (L); The top of the Y shape contains the variable region (V), also known as the fragment antigen-binding (F(ab)) region. The antibody base consists of constant domains (C) and forms the fragment crystallizable region (Fc). Each heavy chain has two regions: constant (CH) and variable (VH). The constant region is identical in all the same isotype antibodies but differs in antibodies of different isotypes. Heavy chains γ, α, and δ have a constant region composed of three tandem Ig domains - CH1, CH2, CH3 - and a hinge region for added flexibility. The "CH" domains in the context of the IgG are as follows: "CHI" refers to positions 118-215 according to EU numbering. "CH2" refers to positions 231-340 according to EU numbering, and "CH3" refers to positions 341-446 according to EU numbering.

Preferably, a recombinant polypeptide according to the invention has the following formula (I):

[Modified Fc region] - [Linker]x - [Ligand binding domain]

wherein:
- the [modified Fc region] is selected from the different embodiments of the modified Fc region and/or the modified CH2 domain of the modified Fc region, as defined in the present description,
- X is an integer equal to 0 or 1;
- [Linker] comprises a polypeptide sequence having 1 to 100 amino acids;
- [Ligand binding domain] comprises a sequence capable of binding to a target ligand, such as a target substance, a target compound, a target molecule or even a target cell, (i) for example a ligand binding domain which is capable of binding to a specific antigen of a target cell, or a sequence capable of binding to a natural or tumor antigen, such as the antigen binding domain of an antibody, which is capable of binding to said target or (ii) for example a ligand binding domain which is capable of binding to a cell receptor or binding to the antigen binding domain of an antibody, in which case the ligand binding domain comprises, or consists of, an antigen recognized by the antigen binding domain of said antibodies.

In the context of the present invention, "Fc region" means all or part of an antibody Fc fragment, or "fragment crystallizable region (Fc region)", which generally consists of the heavy chain constant portion beyond the hinge portion, comprising a CH2 and CH3 domain, that is, respectively, "heavy chain constant domain 2" and "heavy chain constant domain 3". Also, this term encompasses the last two constant regions of IgA, IgD and IgE-type immunoglobulins, the last three constant regions of IgM and IgE-type immunoglobulins as well as the N-terminal hinge portion of said regions. Particularly, it means all or part of an Fc fragment of a human IgG-type antibody, and most particularly of an IgG1-type antibody.

The terms "Fc," "Fc-containing protein" or "Fc-containing molecule" as used herein refer to a monomeric, dimeric or heterodimeric protein having at least an immunoglobulin CH2 and CH3 domain. The CH2 and CH3 domains can form at least a part of the dimeric region of the protein/molecule (e.g., antibody).

According to a preferred embodiment, the recombinant polypeptide is an antibody or a fragment thereof, and the modification in the CH2 domain is symmetrical or asymmetrical, with respect to the pair of CH2 domains constituting the antibody.

In the most preferred embodiment, the CD16+ cell is a NK cell or a neutrophil.

Preferably, in this embodiment, the CD16+ EVs are NK cell-derived EVs (NK-EVs), or, where applicable, any precursor cell-derived EVs. The NK (Natural Killer) cells or any precursor cells, from which the CD16+ EVs are derived from, are preferably allogeneic or autologous with respect to an individual in need thereof.

A NK cell is a lymphocyte capable of spontaneously destroying target cells, involving MHC class 1 molecules. The specificity of these NK cells is to be capable of lysing diseased cells without requiring prior activation and without contacting the pathogenic agent.

The NK cells are found in many tissues and can be obtained, for example, from the lymph nodes, spleen, liver, lungs, intestines, decidua and can also be obtained from iPS cells or from embryonic stem cells (ESC). Generally, cord blood, peripheral blood, mobilized peripheral blood and bone marrow, which contain heterogeneous lymphocytic cell populations, are used to provide a large number of NK cells for research and clinical use. The NK cells of the present invention can be derived from any source comprising such cells, including NK cell precursors.

Methods for selecting NK cells from blood, bone marrow or tissue samples are well known in the field (US5770387). The most commonly used protocols are based on the isolation and the purification of CD56+ cells, generally after fractioning mononuclear cells, and the depletion of non-NK cells such as CD3+, CD34+, CD133+, etc. Combinations of two or more protocols can be used to provide NK cell populations having a greater purity with respect to non-NK contaminants. Kits available on the market for isolating NK cells comprise one-step procedures (for example, CD56 microbeads and kits for isolating CD56+, CD56+ /CD16+ from Miltenyi Biotec, Auburn CA), and procedures in several steps, including depletion, or partial depletion, of CD3+ or depletion with non-NK cell antibodies recognizing and removing T-cells (for example, OKT-3), B- cells, stem cells, dendritic cells, monocytes, granulocytes and erythroid cells. Therefore, in certain implementations, the NK cells are screened for CD45+/CD56+ CD3, CD45+/CD56+ /CD37CD16+, CD45+/CD56+/CD3VCD16, preferably CD45+/CD56+/CD3VCD16+.

In a preferred embodiment, the CD16+ EVs is neutrophil-derived EVs. The human IgG Fc receptor CD16 (FcγRIII) consists of two isoforms (CD16a/FcγRIIIa and CD16b/FcγRIIIb) that are encoded by two highly homologous genes. While CD16a is a membrane-spanning protein, CD16b is linked to the plasma membrane via a GPI anchor. CD16b is expressed only by neutrophils and it primarily recognizes IgG-containing immune complexes, providing an important link between innate and adaptive immunity.

A second object of protection is constituted by a method of obtaining at least one EV defined in any one of claims 1 to 9, the method comprising:
(i) incubating the recombinant polypeptide with the CD16+ cell or with the EV derived from a CD16+ cell, for forming a complex;
and wherein, if the incubating step (i) is carried out with the CD16+ cell, the method further comprises:
(ii) collecting at least one EV complexed to the recombinant polypeptide.

In the context of the invention, a CD3-depleted UCBMCs is used as a precursor to produce activated and expanded NK cells (eNK) as described herein. Obtaining a population of CD56+ CD3- NK cells with CD16 expression is a first crucial step for the success of this method. Authors obtained a population of -98% CD3-CD56+ NK cells, in which CD16 was present in the 60%-90% of the cells of the population (Fig. 1) (Villalba M, et al. Research Square. 2024). These eNK cells expressed high levels of the activation marker CD69 and the natural cytotoxicity receptors (NCR) NKp30, NKp44 and NKp46.

The EVs derived from said eNK cells, which are obtained by an in vitro method also disclosed herein, are expressing proteins that were never found before on the EV membrane, such as CD16. It is the first time that the cd16 expression on NK-derived EVs has been documented. The in vitro method of obtaining the claimed EVs is used to load such EV with a Fc-modified antibody which displays high affinity for CD16. Although the quantification of the CD16 on EVs was not possible due to technical challenges, the fact that the Fc modified Ab is able to be effectively loaded onto EVs, tells us that surface CD16 concentration is sufficient. The binding of the CD16 membrane protein with the specific Ab through the Ab Fc modified domain results in what we termed "armed EVs" (Villalba M, et al. Research Square. 2024; Federici C, et al. Front Immunol 2020. Lugini L, et al. J Immunol. 2012).

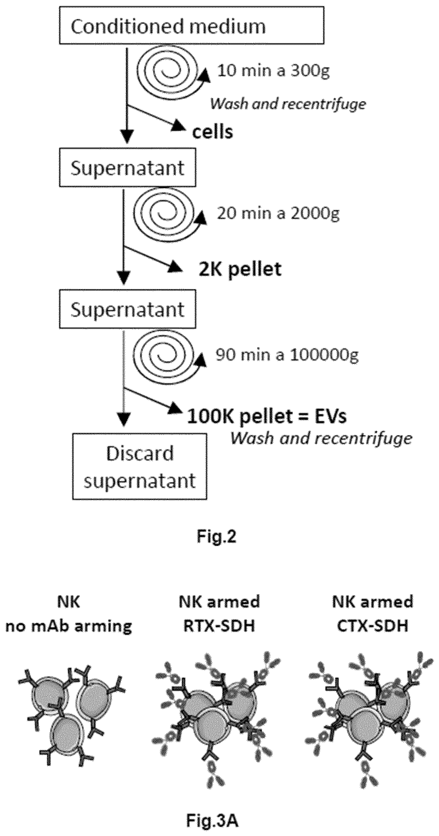

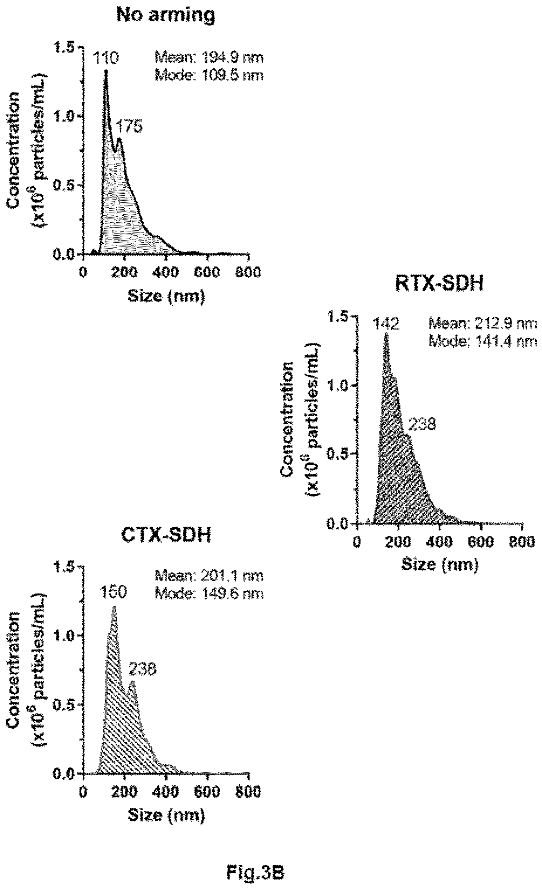

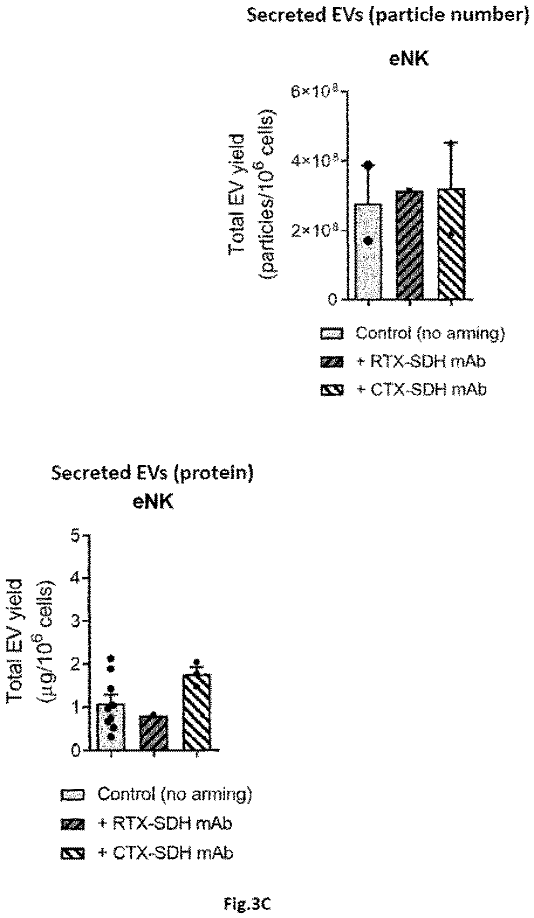

In a preferred embodiment, cells are armed with SDH-modified mAbs, and subsequently cultured in EV-free medium, in order to obtain only EVs derived from cultured NK cells (Figure 3). After 48 h of incubation, the conditioned media (CM) was cleared from these cultures by serial centrifugation and NK-derived EVs were obtained by ultracentrifugation (Figure 2). Nanoparticle tracking analysis of NK-EV isolated samples showed a median size between 100-150 nm, most of them being in the range of 70-250 nm. The NK-derived EVs resulting from this method, are armed with the SDH-modified mAb which the cells are culture in the EV-free medium.

In another particular embodiment, cells are cultured in EV-free medium, in order to obtain only EVs derived from cultured NK cells (Figure 2). After 48 h of incubation, the conditioned media (CM) is cleared from these cultures by serial centrifugation and NK-derived EVs are obtained by ultracentrifugation. The NK EVs resulting from this obtention method are not armed with any mAb. Once the NK-EVs are isolated, NK-EVs are resuspended and incubated with SDH-modified mAbs in order to obtain the armed NK-EVs.

When NK-EVs are obtained from NK cells cultured not armed with any mAb, these EVs are not armed and can be obtained by ultracentrifugation and used immediately, or frozen at -80°C for long-term storage. After protein quantification, NK-EVs can be thawed on ice for 30 min, resuspended in PBS buffer at 100 µg/mL total protein concentration, and incubated with SDH-modified mAbs at 37°C for 1 h to perform NK-EV arming. After the incubation, armed NK-EVs can be ultracentrifuged to remove excess of mAbs and resuspended in the desired solution (PBS or culture media) for further use.

In addition, regardless of the method and duration of storage, there is a decrease in quality while frozen. Freezing the sample could induce both proteolysis and aggregation/non-specific binding to the CD16-SDHmAB. In the experiments presented herein, previously frozen samples were always used to obtain NK-EVs, increasing the risk that ultracentrifugation disrupts the CD16-SDHmAB interaction. In fact, it has been observed a loss of SDHmAb on the eNK after freezing/thawing cycle. Surprisingly, this loss of SDHmAb on NK-EVs was never observed. In cells, probably there is a process of CD16a shaving that blocks arming after thawing. This is not the case for the NK-EVs, making the NK-derived EV population unexpectedly more efficacious due to the better levels of remaining antibody (Miernyk, Thelen. Plant J. 2008).

A third object of protection is the use in medicine of the EV according to any of claims 1 to 9.

Preferably, the EV is a CD16+ EVs as defined herein, for use in medicine.

According to some embodiments, the EV is used in medicine for treating or preventing one disease selected from the group consisting of: cancer, autoimmune disease, and an infectious disease in an individual in need thereof.

In a preferred embodiment, the EV is the CD16+ EVs as defined herein, used for treating or preventing a cancer, an autoimmune disease, or an infectious disease in an individual in need thereof.

In the context of the present invention, any product defined for use in medicine, or for a specific treatment or prevention of illnesses should be understood also as a method of treatment for, and even as a compound for manufacturing a medicament for treating illnesses. The terms "treating", "treatment", "therapy" or "therapeutic" refer to the administration or consumption of an active ingredient, that is an armed NK-EV according to the invention, or of a pharmaceutical composition comprising such an active ingredient for the purposes of curing, relieving, reducing or attenuating, or improving a disease or a pathological disorder, or one or several associated symptoms, or for preventing or slowing down the progression of this symptom or symptoms or this disease, or for stopping the development of this symptom or symptoms, or this disease or this pathological disorder, in a statistically significant manner. More particularly, "treating" or "treatment" includes any approach for obtaining a beneficial effect or a desired result with respect to a disease in an individual. The beneficial or desired clinical results can include, but are not limited to, the attenuation or improvement of the disease or one or several symptoms of such a disease; the diminution or reduction of the extent of the disease, the stabilization, that is, the absence of worsening of a disease, or one or several symptoms of such a disease; the prevention of a disease, or one or several symptoms of such a disease; the prevention of the propagation of a disease, or one or several symptoms of such a disease; the slowing down of a disease, or one or several symptoms of such a disease or the progression of one or more symptoms of such a disease; the diminution of the recurrence of an associated disease, or one or several symptoms of such a disease; and the interruption of a disease, or one or several symptoms of such a disease. In other words, "treatment" as used herein comprises any recovery, improvement, reduction or interruption of a disease, or one or several symptoms of such a disease. A "reduction" of a symptom or a disease means a diminution of the severity or the frequency of the disease or the symptom, or the elimination of the disease or the symptom.

Preferably, the ligand binding domain of a recombinant polypeptide armed to the EVs is capable of recognizing and binding to specific antigens on tumors. The tumors to be treated or prevented are selected from the group but not limited to: melanoma, kidney cancer, prostate cancer, breast cancer, colon cancer, ovarian cancer, lung cancer, bone cancer, pancreatic cancer, skin cancer, head or neck cancer, skin or intraocular malign melanoma, uterine cancer, rectal cancer, anal cancer, stomach cancer, testicular cancer, uterine cancer, Fallopian tube carcinoma, endometrial carcinoma, cervical carcinoma, vaginal carcinoma, vulvar carcinoma, Hodgkin's disease, non-Hodgkin's lymphoma, esophageal cancer, small intestine cancer, endocrine system cancer, thyroid cancer, parathyroid cancer, adrenal gland cancer, soft tissue sarcoma, urethral cancer, penile cancer, chronic or acute leukemias including acute myeloid leukemia, chronic myeloid leukemia, acute lymphoblastic leukemia, chronic lymphocytic leukemia, childhood solid tumors, lymphoid lymphoma, bladder cancer, kidney or urethral cancer, renal pelvis carcinoma, central nervous system (CNS) neoplasm, CNS primary lymphoma, tumor angiogenesis, spinal tumor, brainstem glioma, pituitary adenoma, Kaposi's sarcoma, epidermoid cancer, squamous cell cancer, T-lymphocyte lymphoma, environmentally-induced cancers and any combinations thereof.

According to another particular embodiment, the ligand binding domain of a recombinant polypeptide armed to the EVs is capable of recognizing and binding to infectious disease antigens on the surface of microorganisms, pathogens, or pathogen infected cells. Those infectious disease antigens appear in the patient under an infectious disease condition, being that disease selected from the group of diseases, but not limited to: (a) a disease selected from flu, herpes, giardiasis, malaria and leishmaniasis; (b) a pathogen infection with a virus selected from human immunodeficiency virus (HIV), hepatitis virus, herpes virus, adenovirus, influenza virus, flavivirus, echovirus, rhinovirus, coxsackievirus, cornovirus, respiratory syncytial virus, mumps virus, rotavirus, measles virus, rubella vims, parvovirus, vaccinia virus, HTLV virus, dengue virus, papillomavirus, molluscum virus, poliomyelitis virus, rabies virus, JC virus and arboviral encephalitis virus; (c) a pathogen infection with a bacterium selected from Chlamydia, rickettsia bacteria, mycobacteria, staphylococcus, streptococcus, pneumococcus, meningococcus and gonococcus, Klebsiella, Proteus, Serratia, Pseudomonas, Legionella, Diphtheria, Salmonella, Bacilli, cholera, tetanus, botulism, carbon disease, plague, leptospirosis and Lyme disease bacteria; (d) a pathogen infection with a fungus selected from Candida, Cryptococcus neoformans, Aspergillus, Genus Mucorales, Sporothrix schenckii, Blastomyces dermatitidis , Paracoccidioides brasiliensis, Coccidioides immitis and Histoplasma capsulatum ; or (e) a pathogen infection with a parasite selected from Entamoeba histolytica, Balantidium coll, Naegleria fowleri, Acanthamoeba sp., Giardia lamblia, Cryptosporidium sp., Pneumocystis carinii, Plasmodium virax, Babesia microti, Trypanosoma brucei, Trypanosoma cruzi, Leishmania donovani, Toxoplasma gondii and Nippostrongylus brasiliensis.

According to another particular embodiments, the ligand binding domain of a recombinant polypeptide armed to the EVs is capable of recognizing and binding to infectious disease antigens on the surface of microorganisms, pathogens, or pathogen infected cells. Those infectious disease antigens appear in the patient under an infectious disease condition, being that disease selected from the group of diseases, but not limited to: organ graft rejection, graft-versus-host disease, rheumatoid arthritis, systemic lupus erythematosus, scleroderma, primitive Sjogren's syndrome (or Gougerot-Sjogren's syndrome), autoimmune polyneuropathies such as multiple sclerosis, type-I diabetes, autoimmune hepatitis, ankylosing spondylitis, Reiter's syndrome, gouty arthritis, celiac disease, Crohn's disease, Hashimoto's thyroiditis, Addison's disease, autoimmune hepatitis, Basedow's disease, ulcerative colitis, vasculitis such as antineutrophil cytoplasmic antibody (ANCA)associated systemic vasculitis, autoimmune cytopenia and other hematologic complications in adults and children, such as acute or chronic autoimmune thrombopenia, autoimmune hemolytic anemias, hemolytic disease of the newborn (HDN), cold agglutinin disease, thrombotic thrombocytopenic purpura and autoimmune acquired hemophilia, Goodpasture syndrome, extramembranous nephropathy, autoimmune oily skin conditions, refractory myasthenia, mixed cryoglobulinemia, psoriasis, juvenile idiopathic arthritis, inflammatory myositis, dermatomyositis and systemic autoimmune conditions in children including antiphospholipid syndrome.

A fourth object of protection is a composition comprising at least one EV defined in any one of the claims 1 to 9. In a preferred case, the composition is a pharmaceutical composition. Also preferably, the pharmaceutical composition comprises an excipient or a pharmacologically acceptable vehicle.

As can be seen from the above statement, a composition is also provided, preferably being a pharmaceutical composition. The composition can comprise one or more CD16+ EVs complexed to recombinant polypeptides (armed NK-EVs) as disclosed herein, formulated with a pharmacologically or pharmaceutically acceptable excipient or a vehicle. This composition can comprise one or several combinations of NK-EVs armed with recombinant polypeptides according to the invention (for example, two or more different ones). For example, a pharmaceutical composition described herein can comprise a combination of armed NK-EVs which bind to different epitopes on the target antigen or antibodies having complementary activities (or immunoconjugates or bispecific compounds).

A pharmaceutical composition must generally be sterile and stable under manufacturing and storage conditions. The composition can be formulated in the form of a solution, microemulsion, solution, microemulsion, liposome or other ordered structure suited for high medicament concentration. Besides, the pharmaceutical compositions according to the invention can be administered according to one or several methods known in the art, by one or several routes of administration. As will be appreciated by those skilled in the art, the route of administration and/or the mode of administration will vary depending on the desired result. According to a preferred embodiment, the routes of administration for the armed NK-EVs according to the invention comprise the administration by intravenous, intramuscular, intradermal, intraperitoneal, subcutaneous, spinal route or any other parenteral route of administration such as injection or perfusion.

The armed NK-EVs according to the invention can also be administered by a topical, epithelial or mucosal route of administration, for example a non-parenteral route, such as intranasal, oral, vaginal, rectal, sublingual or topical.

The composition can comprise armed NK-EVs in an amount selected from the group consisting of: at least 1 mg/ml, 5 mg/ml, 10 mg/ml, 50 mg/ml, 100 mg/ml, 150 mg/ml, 200 mg/ml, 1-300 mg/ml and 100-300 mg/ml of armed NK-EVs.

The pharmaceutical compositions described herein can also be administered in combination therapy, that is, in combination with other agents. For example, the combination therapy can include an armed NK-EVs described herein in combination with at least another antipathogenic agent.

In particular cases, the pharmaceutical compositions described herein can include other compounds, agents and/or medicaments used for treating pathologies such as a cancer, an autoimmune disease and derivatives thereof or an infectious disease. These compounds, agents and/or medicaments can include but not limited to, for example, chemotherapeutic agents, small molecule agents or antibodies stimulating an immune response against a given cancer.

The pharmaceutical compositions described herein can comprise one or several pharmaceutically acceptable salts. "Pharmaceutically acceptable salt" means a salt conserving the desired biological activity of the parent compound and not transmitting undesirable toxic effects. Acid addition salts and base addition salts are examples of salts. The acid addition salts comprise non-toxic inorganic acids such as hydrochloric acid, nitric acid, phosphoric acid, sulfuric acid, hydrobromic acid, hydroiodic acid, phosphoric acid and mono- and di-carboxylic aliphatic acids, phenyl-substituted alkanoic acids, hydroxy alkanes. Salts derived from non-toxic organic acids such as acids, aromatic acids, aromatic and aliphatic sulfonic acids are included. The base addition salts comprise, for example, alkaline earth metals such as sodium, potassium, magnesium and calcium, as well as N, N'- dibenzylethylenediamine, N-methylglucamine, chloroprocaine, choline, diethanolamine, ethylenediamine, procaine and other. Salts derived from toxic organic amines are included.

The pharmaceutical compositions described herein can also include a pharmaceutically acceptable antioxidant. Examples of pharmaceutically acceptable antioxidants comprise (1) water-soluble antioxidants such as ascorbic acid, cysteine hydrochloride, sodium bisulfate, sodium metabisulfite, sodium sulfite, etc., (2) oil-soluble antioxidants, oxidizing agents such as ascorbyl palmitate, butylated hydroxyanisole (BHA), butylated hydroxytoluene (BHT), lecithin, propyl gallate, alpha- tocopherol and others; and (3) metal chelators such as citric acid, ethylenediaminetetraacetic acid (EDTA), sorbitol, tartaric acid, phosphoric acid and others are included.

Examples of suitable aqueous or non-aqueous carriers that can be used in the pharmaceutical compositions described herein comprise water, ethanol, polyols (for example, glycerol, propylene glycol, polyethylene glycol, etc.) and suitable mixtures thereof, vegetable oils such as olive oil and injectable organic esters such as ethyl oleate. Suitable flowability can be maintained, for example, conserving the required particle size in the case of a dispersion and using surfactants. These compositions can also contain adjuvants such as preservatives, wetting agents, emulsifying agents and dispersing agents. The prevention of the presence of microorganisms can be ensured by both the sterilization methods described hereinafter and the inclusion of several antibacterial and antifungal agents such as parabens, chlorobutanol, phenol, sorbic acid and others. It can also be desirable to include isotonic agents, such as sugars, sodium chloride, in the composition. Furthermore, the inclusion of absorption-retardant agents, such as aluminum monostearate and gelatin, can delay the absorption of injectable pharmaceutical forms.

The active compounds can be prepared with carriers that will protect the compound against a fast release, such as a controlled release formulation, including implants, transdermal patches and microencapsulated delivery systems. Biodegradable and biocompatible polymers can be used, such as ethylene-vinyl acetate, poly anhydrides, polyglycolic acid, collagen, polyorthoesters and polylactic acid. Many methods for preparing these formulations are patented or generally known to the skilled persons. Pharmaceutically acceptable "carriers" or "vehicles" comprise sterile aqueous solutions or dispersions and sterile powders for the extemporary preparation of sterile injectable solutions or dispersions. The use of such carriers or vehicles and agents for pharmaceutically active substances is well known in the field. Except where a conventional medium or agent is not compatible with the active ingredient, the use thereof in the pharmaceutical compositions described herein is contemplated. Complementary active substances can also be incorporated in the compositions. The carrier can be a solvent or a dispersion medium containing, for example, water, ethanol, polyol (for example, liquid glycerol, propylene glycol and polyethylene glycol, and others), and suitable mixtures thereof. Suitable flowability can be maintained, for example, by use of a coating such as lecithin, by conservation of the required particle size in the case of a dispersion and by the use of surfactants. In many cases, it will be preferable to include isotonic agents, for example sugars, polyalcohols such as mannitol and sorbitol, or sodium chloride in the composition. Delayed absorption of the injectable compositions can be brought about by the inclusion of an absorption- retardant agent, for example, monostearate salts and gelatin, in the composition.

The sterile injectable solutions can be prepared including the active compound, that is, armed NK-EVs, in a required amount in a suitable solvent, potentially with one or a combination of ingredients listed hereinafter, then sterilizing by means of microfiltration. Generally, the dispersions are prepared incorporating the active compound in a sterile vehicle containing a basic dispersion medium and other necessary ingredients from those listed hereinafter. In the case of sterile powders for the preparation of sterile injectable solutions, the preferred preparation method is a method of drying under vacuum, wherein an active ingredient powder plus any other desired ingredient is prepared from the previously sterilized and filtered solution, and lyophilizing.

The amount of active ingredient which can be combined with carrier materials to prepare a unit dosage form can vary according to the subject to be treated and the particular mode of administration. In the context of the present invention, a unit dosage form designates a physical unit which is convenient as a unit dose for the individual to be treated; each unit, associated with the required pharmaceutical carrier, causes the desired therapeutic effect.

The amount of active ingredient, that is, armed NK-EVs, which can be combined with a carrier material to prepare a unit dosage form, will generally be the amount of the composition which causes a therapeutic effect. According to certain embodiments, the amount of active ingredient, that is, armed NK-EVs, is about 0.01% to about 99% with respect to the final composition amount, preferably about 0.1% to about 70% with respect to the final composition amount, most of the time in combination with a pharmaceutically acceptable carrier.

Dosage schedules are adjusted in order to obtain the desired optimal response (for example, a therapeutic response). According to certain embodiments, a single bolus administration is possible and several divided doses can be administered over a long period of time, or the dose can be proportionally reduced or increased as indicated in a situation of imminent treatment. Preferably, the formulation of parenteral compositions is in the form of unit doses, specifically in order to facilitate administration and dosage uniformity.

Preferably, a treatment regimen for the administration of the pharmaceutical composition can be one administration once a week, once every two weeks, once every three weeks, once every four weeks, once a month, once every three months or once every three months to six months.

Preferably, the dosage schedules for the administration of the pharmaceutical compositions according to the invention comprise 1 mg/kg of body weight or 3 mg/kg of body weight per intravenous administration. Specifically, the composition is administered (i) once every 4 weeks, followed by (ii) once every 3 months.

In a preferred embodiment, two or more types of armed NK-EVs having different binding specificities are administered simultaneously, in which case the dose of each armed NK-EV administered is within the indicated ranges. The armed NK-EVs are generally administered several times. The interval between the unit doses can be, for example, once a week, once a month, every three months or once a year. In addition, the intervals can be irregular. In certain embodiments, the dosage is adjusted in order to obtain an armed NK-EV plasma concentration of about 1-1000 pg/ml, and in certain embodiments, an armed NK-EV plasma concentration of about 25-300 pg/ml.

The armed NK-EVs can be administered in the form of a sustained release formulation, in which case less frequent administration is required. Dosage and frequency vary depending on the armed NK-EVs half-life in the patient.

According to certain embodiments, the pharmaceutical composition is used for a prophylactic or therapeutic treatment. Dosage and frequency of administration can vary based on whether the treatment is prophylactic or therapeutic. In the prophylactic applications, relatively weak doses are administered over long periods at relatively infrequent intervals. Certain patients continue to receive treatment for the rest of their life. In therapeutic applications, relatively high doses at relatively short intervals can be required until progression of the disease is reduced or stopped, preferably until the patient shows a partial or complete improvement of the symptoms of the disease. Thereafter, the patient can be administered a prophylactic regimen.

The real dosage levels of the active ingredients of the pharmaceutical compositions according to the invention disclosed herein, are not toxic for the patient in order to obtain the desired therapeutic response for the individual, particularly the composition and the mode of administration. It is possible to vary same in order to obtain an effective amount of active ingredient. The selected dosage level depends on the particular composition being used, or the activity of its ester, salt or amide, the route of administration, the time of administration, the elimination rate of the particular compound being used, the duration of treatment, other medicaments, compounds and/or substances used in combination with a particular composition, the age, sex, weight, condition, general health and medical history of the individual to be treated, as well as similar factors well known in the medical field. The dosage level can also vary depending on several pharmacokinetic factors.

According to a particular embodiment, a therapeutically effective dose can prevent or delay the onset of a pathology. For example, laboratory tests used to diagnose a disease comprise chemistry, hematology, serology and radiology. Consequently, the clinical or biochemical assays which monitor one of the elements hereinabove can be used to determine if a particular treatment is a therapeutically effective dose for treating the disease. One skilled in the art can determine such amounts depending on factors such as the size of the individual, the severity of the symptoms of the individual, and the particular composition or the route of administration selected.

The therapeutic composition can be administered by means of medical devices known in the field. In certain cases, the armed NK-EVs according to the invention can be formulated to ensure good in vivo distribution. For example, the blood-brain barrier (BBB) excludes many highly hydrophilic compounds. To ensure that the therapeutic compounds according to the invention go through the BBB (if so desired), they can be formulated, for example, in liposomes. The liposomes can contain one or several substances which are selectively transported to specific cells or organs, and therefore can improve the targeted administration of medicaments.

### Brief description of drawings

**Figure 1****,** represents the protocol followed to expand eNK cells. This work benefited from umbilical cord blood units (UCBs). NK cells were expanded as previously described. Briefly, UCBMCs or PBMCs were isolated through density gradient centrifugation using Histopaque-1077 (Sigma). Blood samples were diluted at 1:1 ratio with RPMI then layered above 10 mL Histopaque in a 50 mL conical tube. Once centrifuged for 30 min at 400×g, the white layers of mononuclear cells (MCs) were collected and washed. Using EasySepTM Human CD3 Positive Isolation kit (StemCell Technologies), the CD3+ cell fraction (T and NKT cells) of the MCs was depleted in each sample to better culture the NK cells. Once depletion was verified through flow cytometry, cells were cultured for 20 days. NKs were cultured with γ-irradiated PLH at a 1:4 (NK cell: feeder cell) ratio and IL-2 (100 IU/mL) and IL-15 (5 ng/mL). Feeder cells and cytokines were refreshed every 3-4 days. To monitor expansion, NK cells were stained with APC-labelled anti-CD3 mAb and PE or Vio770-labelled anti-CD56 mAb (both form BD Biosciences). Culture viability was determined at regular intervals through flow cytometry analysis.
**Figure 2** represents the method to obtain NK cell-derived EVs. **Graph 2A.** Conditioned media was recovered and after several centrifugations to remove cells, dead cells and cell debris, EVs were obtained by ultracentrifugation at 100,000 × g for 2 h at 4°C.
**Figure 3** represents the characterization of mAb-armed NK-derived EVs. In Figure 3, it is shown: **Fig. 3A****.** UCB-expanded NK cells (eNK) were armed with SDH-modified Rituximab (RTX-SDH, blue) or Cetuximab (CTX-SDH, yellow) and incubated in EV-free RPMI 10% FBS for 48h. **Fig. 3B****.** Size and concentration of NK-EVs control (top, gray, no stripes), armed with RTX-SDH (middle, blue, right leaning diagonal stripes) and armed with CTX-SDH (bottom, gold, left leaning diagonal stripes) was determined by nanoparticle tracking analysis (NTA). Numbers over the histograms indicate size in nanometers of relevant peaks. **Fig. 3C****.** Graphs show total EV yield for eNK cells, expressed as particles/million cells cultured (from NTA) and as µg of total protein/million cells (from BCA quantification). Size and concentration of NK-EVs control (left, gray, no stripes), armed with RTX-SDH (middle, blue, right-leaning diagonal stripes) and armed with CTX-SDH (right, gold, left-leaning diagonal stripes) was determined by nanoparticle tracking analysis (NTA). Graphs show total EV yield for eNK cells, expressed as particles/million cells cultured (from NTA) and as µg of total protein/million cells (from BCA quantification).

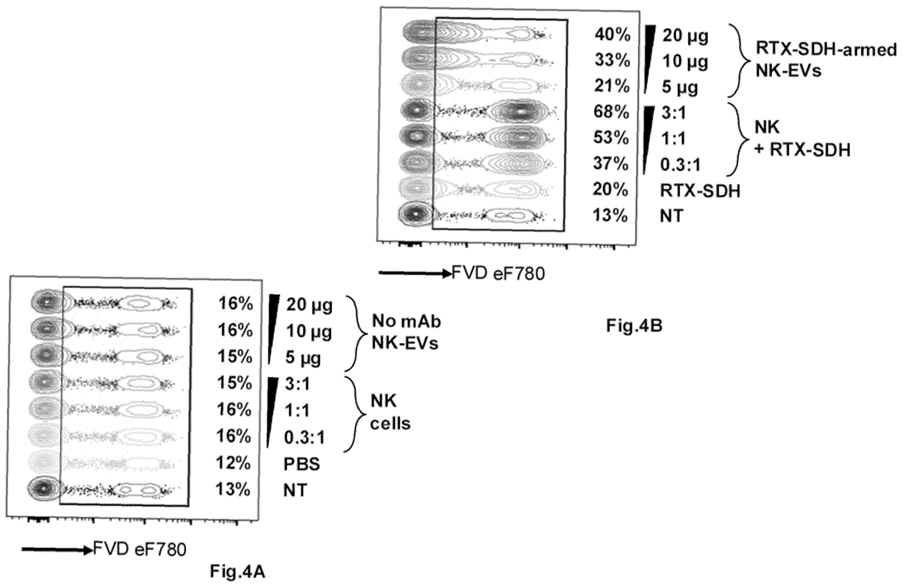

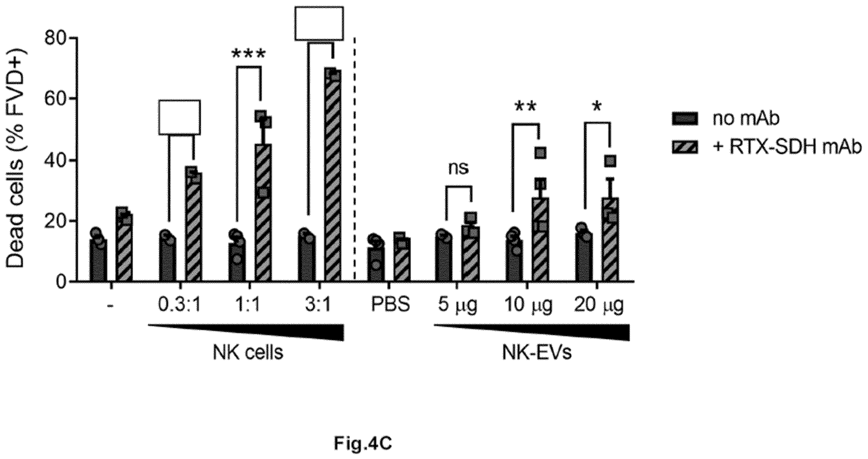

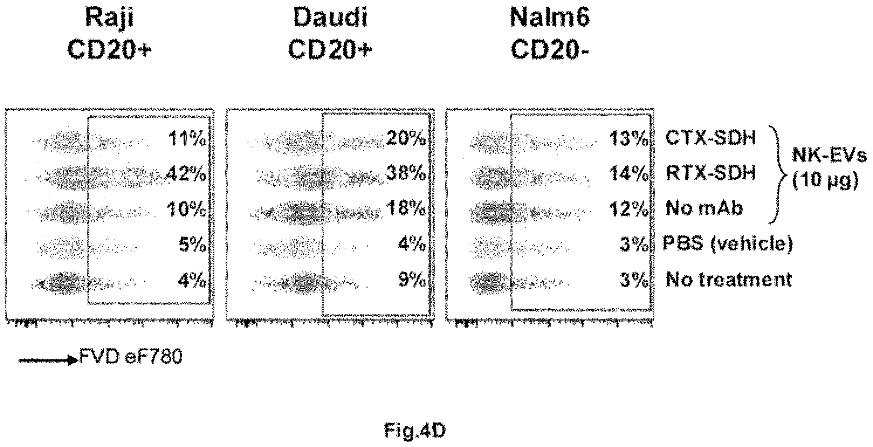

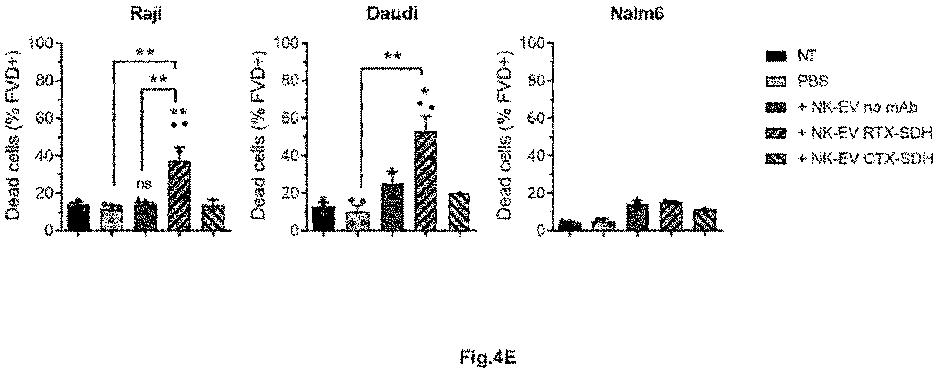

**Figure 4** represents the specificity of the RTX-armed NK-EVs. Hematological cancer cell lines Raji and Daudi (both CD20+) and Nalm6 (CD20) were stained with CellTrace^{™} CFSE, and treated with NK-EVs from control (no mAb) or mAb-armed NK cells. After 24 h, cells were stained for cell death (FVD^{™} eF780) and CFSE+ target cells were gated and analyzed by FACS. In this Figure 4, it is shown: **Fig. 4A****.** Percentage of Raji cell death after co-culture with NK cells at different increasing effector: target ratios (from 0.3:1 to 3:1), or treatment with increasing amounts (5 to 20 µg) of control (no mAb) NK-EVs. **Fig. 4B****.** Percentage of Raji cell death after co-culture with NK cells in the presence of RTX-SDH mAb (ADCC), or treated with RTX-armed NK-EVs. **Fig. 4C****.** The proportion of dead cells (FVD^{™} eF780+) among target Raji cells after different treatments is depicted in the graph, for conditions with no mAb arming (left, black, no stripes) or RTX-SDH mAb arming (right, blue, right-leaning diagonal stripes). **Fig. 4D****.** Percentage of death cells among CFSE-stained target cells Raji (left), Daudi (middle) and Nalm6 (right) after treatment with vehicle (PBS) or NK-EVs control (no mAb) or NK-EVs derived from RTX-armed (anti-CD20) or CTX-armed (anti-EGFR) NK cells. **Fig. 4E****.** The proportion of dead cells (FVD^{™} eF780+) among target CD20+ Raji (left), CD20+ Daudi (middle) and CD20-negative Nalm6 cells (right) after different treatments is depicted in the graph, for the untreated target cells (black), or treatment with vehicle PBS (light blue, dotted), or with NK-EVs control (red, no stripes), or NK-EVs armed with RTX-SDH (blue, right-leaning stripes) or armed with CTX-SDH (gold, left-leaning stripes). Bars represent mean ± SEM. Statistical significance between conditions was determined by one-way ANOVA (C) or Student's t-Test (E). * p < 0.05; ** p < 0.01; *** p < 0.001; ns = not significant.
**Figure 5** represent the efficacy of SDH-RTX-armed EVs vs. non-armed EVs. Hematological cancer cell lines Daudi (CD20+) and Nalm6 (CD20-) were stained with CellTrace^{™} CFSE, and treated with NK-derived EVs directly armed with mAb. After 24 h, cells were stained for cell death (FVD^{™} eF780) and CFSE+ target cells were gated and analyzed by FACS. In this Figure 5, it is shown: **Fig. 5A** **left.** Percentage of Daudi cell death after treatment with increasing amounts (5 to 20 µg) of control (no mAb) NK-EV. **Fig. 5A** **right.** Percentage of Daudi cell death after treatment with increasing amounts (5 to 20 µg) of NK-EVs directly armed with SDH-modified RTX mAbs. **Fig. 5B****.** The proportion of dead cells (FVD^{™} eF780+) among target Daudi cells after different treatments is depicted in the graph, for conditions with no mAb arming (white, no stripes) or RTX-SDH mAb arming (blue, right-leaning stripes). **Fig. 5C****.** The proportion of dead cells (FVD^{™} eF780+) among target Nalm6 cells after different treatments is depicted in the graph, for conditions with no mAb arming (white, no stripes) or RTX-SDH mAb arming (blue, right-leaning stripes). Bars represent mean ± SEM. Statistical significance between conditions was determined by two-way ANOVA (C-D). * p < 0.05; ** p < 0.01; *** p < 0.001; ns = not significant.
**Figure 6** represent the efficacy of SDH-RTX-armed EVs vs. Wt-RTX-armed EVs, and the efficacy of SDH-armed EVs vs. mAbs. Hematological cancer cell lines Daudi (CD20+) and Nalm6 (CD20-) were stained with CellTrace^{™} CFSE, and treated with NK-derived EVs directly armed with mAb. After 24 h, cells were stained for cell death (FVD^{™} eF780) and CFSE+ target cells were gated and analyzed by FACS. In this Figure 6, it is shown: **Fig. 6A****.** Percentage of Daudi cell death after treatment with 10 µg/mL of mAb alone, or increasing amounts (5 to 20 µg) of wt-RTX-armed NK-EV (left) or SDH-RTX-armed NK-EV (right). **Fig. 6B****.** The proportion of dead cells (FVD^{™} eF780+) among target Daudi cells after different treatments is depicted in the graph, for conditions with no Wt-RTX-arming (light blue, no stripes) or RTX-SDH mAb arming (blue, right-leaning stripes). **Fig. 6C****.** The proportion of dead cells (FVD^{™} eF780+) among target Nalm6 cells after different treatments is depicted in the graph, for conditions with no Wt-RTX-arming (light blue, no stripes) or RTX-SDH mAb arming (blue, right-leaning stripes). **Fig. 6D****.** Percentage of Daudi (left) or Nalm6 (right) cell death after treatment with 10 µg/mL of mAbs alone, or 20 µg of NK-EVs control (no arming), or armed with SDH-RTX or SDH-CTX mAbs. **Fig. 6E****.** The proportion of dead cells (FVD^{™} eF780+) among target Daudi cells (left) or Nalm6 cells (right) after different treatments is depicted in the graph, for the untreated target cells (black), or treatment with RTX-SDH mAb alone (light blue, dotted pattern) or CTX-SDH mAb alone (yellow, triple-dot pattern), or with NK-EVs control (red, no stripes), or NK-EVs armed with RTX-SDH (blue, right-leaning stripes) or armed with CTX-SDH (gold, left-leaning stripes). Bars represent mean ± SEM. Statistical significance between conditions was determined by two-way ANOVA (C-D). * p < 0.05; ** p < 0.01; *** p < 0.001; ns = not significant.

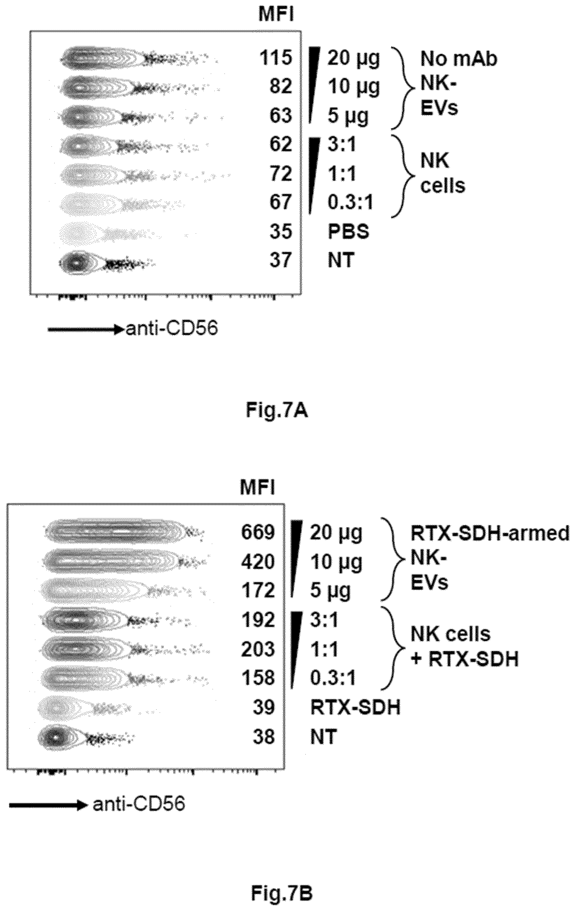

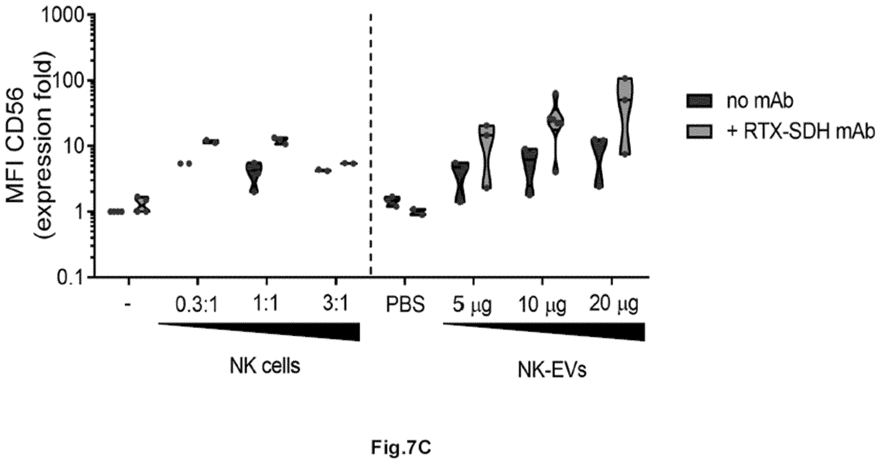

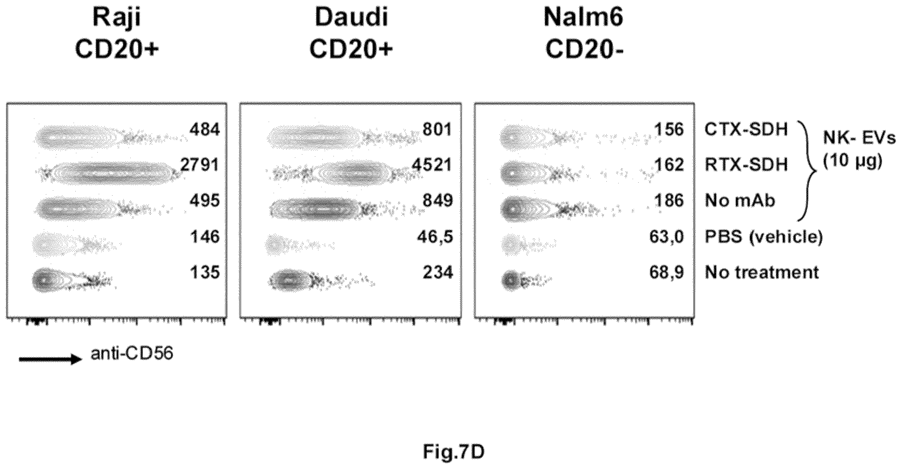

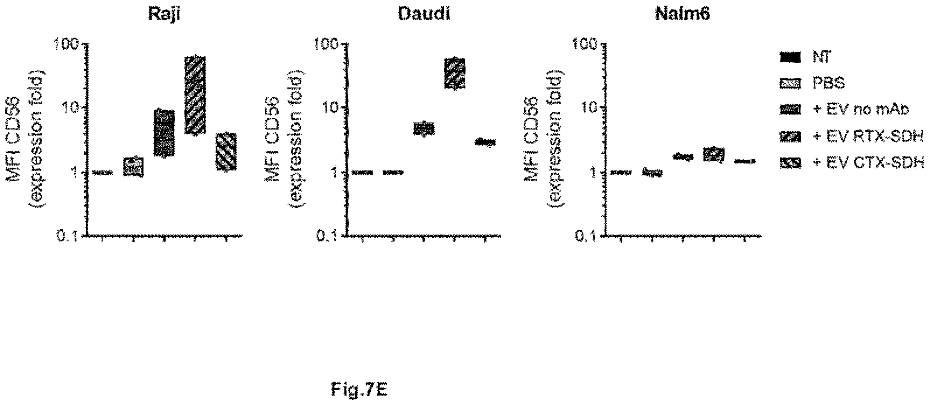

**Figure 7** represents the efficiency contacting the target by surface CD56 detection on NK-EV-treated hematological cancer cells. Hematological cancer cell lines Raji and Daudi (both CD20+) and Nalm6 (CD20) were stained with CellTrace^{™} CFSE, and treated with NK-EVs from control (no mAb) or mAb-armed NK cells. After 24 h, cells were stained with fluorochrome-coupled anti-CD56 antibodies, CFSE+ target cells were gated and analyzed for NK marker CD56 surface expression (median fluorescence intensity or MFI) by FACS. In this Figure 7, it is shown: **Fig. 7A****.** Intensity of CD56 staining on Raji cells after co-culture with NK cells at different increasing effector: target ratios (from 0.3:1 to 3:1), or treatment with increasing amounts (5 to 20 µg) of control (no mAb) NK-EVs. **Fig. 7B****.** Intensity of CD56 staining on Raji cells after co-culture with NK cells in the presence of RTX-SDH mAb (ADCC), or treated with RTX-armed NK-EVs. **Fig. 7C****.** The intensity levels of surface CD56 protein among target Raji cells after different treatments is depicted in the violin graph, for conditions with no mAb (black) or with RTX-SDH mAbs (blue). **Fig. 7D****.** Intensity of CD56 staining on CFSE-stained target cells Raji (left), Daudi (middle) and Nalm6 (right) after treatment with vehicle (PBS) or NK-EVs control (no mAb) or NK-EVs derived from RTX-armed (anti-CD20) or CTX-armed (anti-EGFR) NK cells. **Fig. 7E****.** Intensity of CD56 staining among target CD20+ Raji (left), CD20+ Daudi (middle) or CD20-negative Nalm6 cells (right) after different treatments is depicted in the graph floating bars graph (min to max), for conditions with no treatment (black, first position) or with vehicle PBS (light blue, second position, dotted pattern), or treated with NK-EVs control no mAb (red, third position, no pattern) or NK-EV armed with RTX-SDH mAbs (blue, fourth position, right-leaning diagonal stripes) or with CTX-SDH mAbs (gold, fifth position, left leaning diagonal stripes). Bars represent mean ± SEM.

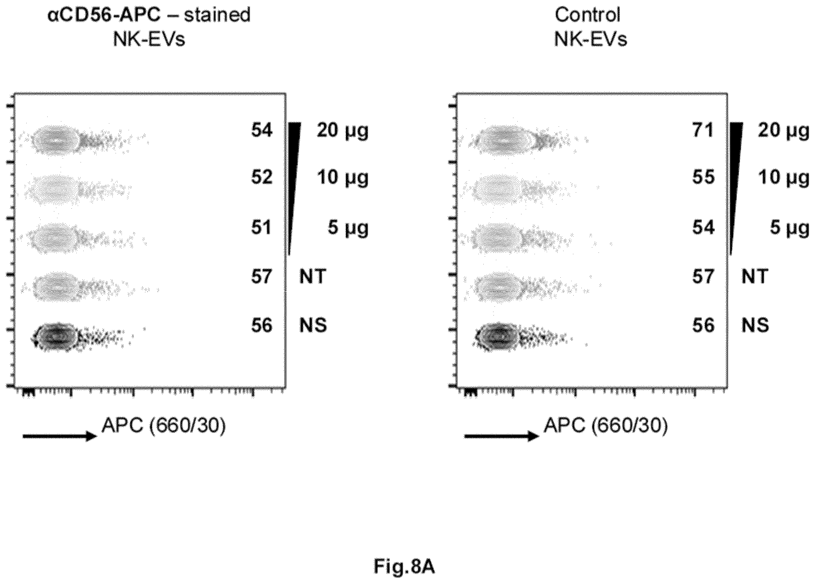

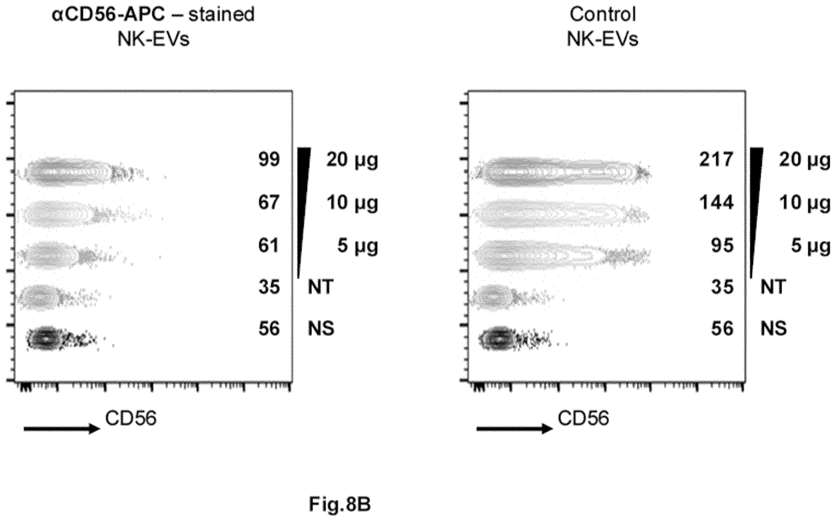

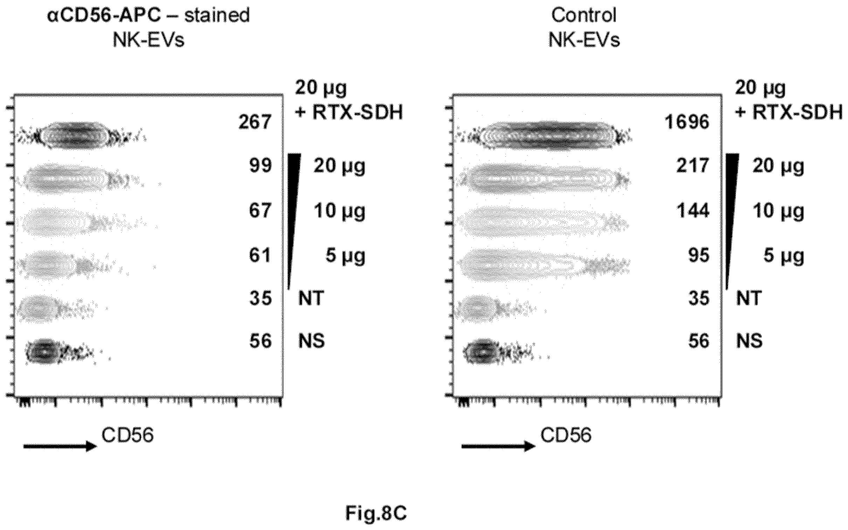

**Figure 8** represents the antibody retention capacity of NK-EVs when binding is through epitope affinity instead of Fc-CD16a interaction (arming). NK cells were stained with allophycocyanin (APC)-coupled anti-CD56 antibodies (αCD56-APC), or not (control), and then they were incubated for NK-EV obtention as described before. Hematological cancer cell line Daudi cells (CD20+) were cultured alone, or treated with EVs from control NK or αCD56-APC-stained NK cells. After 24h, target cells were washed and stained with viability marker and/or fluorochrome-coupled anti-CD56 antibodies, and then cells were gated and analyzed for NK marker CD56 surface expression (median fluorescence intensity or MFI) by FACS. Please, note that all graphs in this figure corresponds to the staining in the target Daudi cells and not to NK-EVs or NK cells. In this Figure 8, it is shown: **Fig. 8A****:** Intensity of APC fluorescence staining on Daudi cells after treatment with increasing amounts (5 to 20 µg) of αCD56-APC-NK-EVs or control (unstained) NK-EVs. The anti-CD56APC has not been transferred because no APC signal in Daudi. **Fig. 8B****:** Intensity of CD56 staining on Daudi cells after treatment with increasing amounts (5 to 20 µg) of αCD56-APC-NK-EVs or control (unstained) NK-EVs. We observed that there is less CD56 when cells were stained with the anti-CD56 antibody. This suggests that having an antibody on the antigen (Ag) probably reduces the possibility of the Ag to be included in the NK-EVs. Hence, in our CD16 arming we should find less CD16, which is not the case. **Fig. 8C****:** Intensity of CD56 staining on Daudi cells in the same conditions as before, but with addition of SDH-RTX at 10 µg/mL It shows that adding the RTX we favor CD56 transfer because the NK-EVs are better upload by Daudi cells. APC fluorescence from this FACS Ab was not present in Daudi treated with these CD56-APC-stained NK-EVs. EVs are washed several times (at least 3) with PBS during the ultracentrifugation, and generally freeze-and-thawed before being used, so it is expectable that antigen-affinity bound mAbs to these EVs could not survive these manipulations.

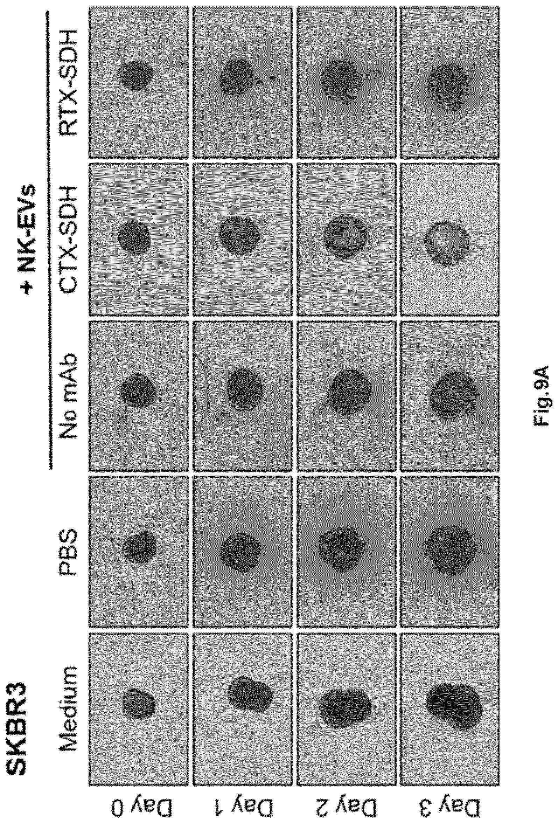

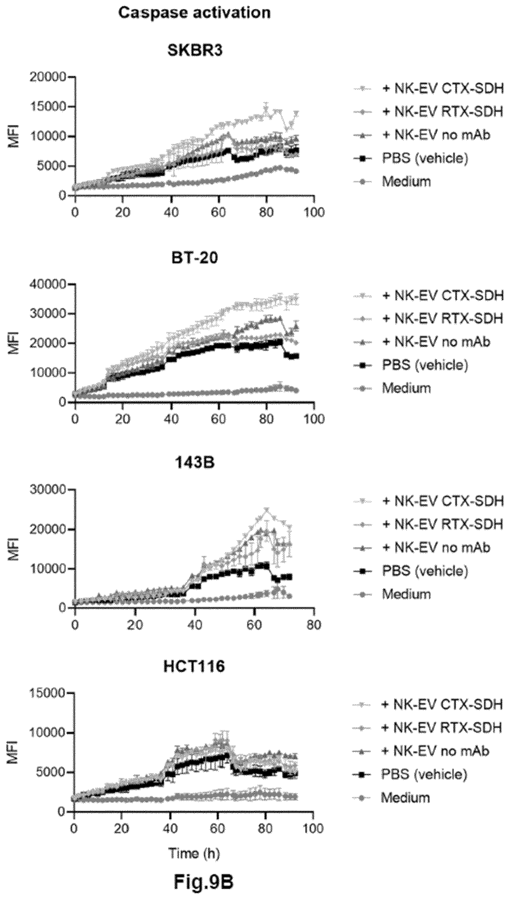

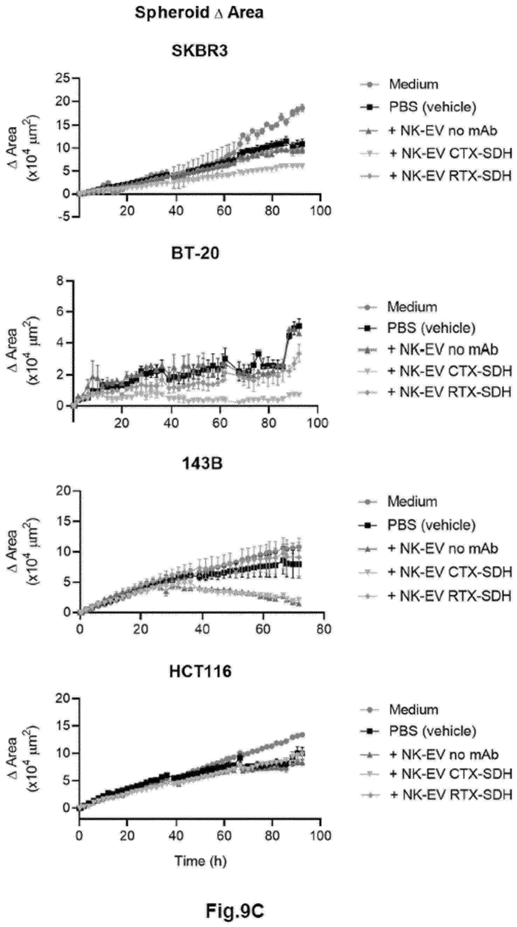

**Figure 9** represents the efficacy of NK-EVs armed with CTX-SDH on EGFR+ tumor cell spheroids. Solid tumor cells from different cell lines were seeded in ultra-low attachment plates to generate tumor spheroids. Then, these spheroids were treated with or not with vehicle (PBS, black squares) or 20 µg of NK-EVs derived from control unarmed NK cells (no mAb, red upward triangle) or armed with CTX (anti-EGFR, gold downward triangle) or RTX (anti-CD20, blue diamond) mAbs, for up to 96 h. Induction of apoptosis of spheroids was followed with CellEvent^{™} Caspase-3/7 Green detection reagent, and spheroid area and green fluorescence was measured every two hours by kinetic imaging using the Cytation 5 platform, and data analysis was performed using Gen5.3 software. In Figure 9, it is shown: **Fig. 9A****.** Representative images of SKBR3 breast cancer cell spheroids obtained by the Cytation 5 at start of experiment (Day 0) and days 1-3 after treatment, depicting green fluorescence that represents caspase-3/7 activity. **Fig. 9B****.** Measurement over time of apoptosis by MFI of green fluorescence of solid tumor cell spheroids under different NK-EV treatments. **Fig. 9C****.** Change of area over time of solid tumor cell spheroids under different NK-EV treatments. For B-C, lines and data points represent mean ± SEM for experiments perform in duplicates.

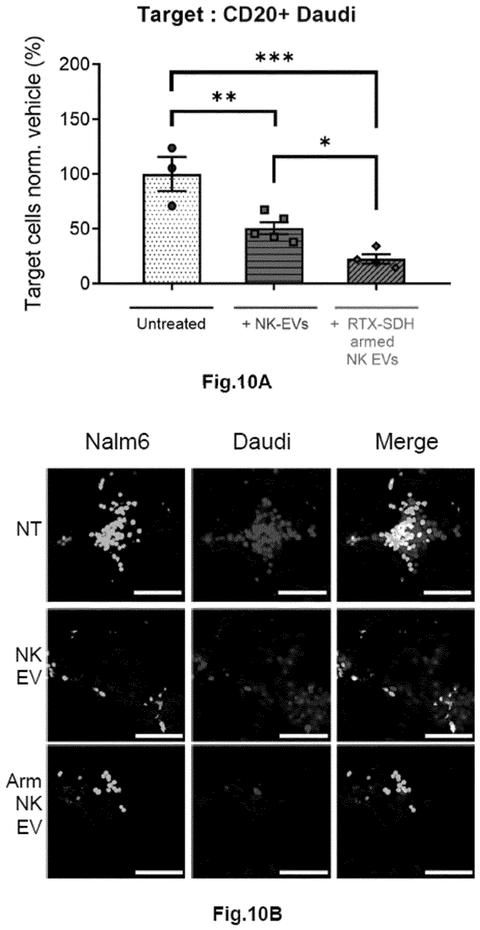

**Figure 10** represents the in vivo cytotoxicity of SDH-RTX-armed NK-EVs. In Figure 10 is shown: **Fig. 10A****.** CTV-stained Daudi cells were intraperitoneally co-injected into NSG mice, and then treated or not with 100 µg of unarmed NK-EVs (control) or armed with RTX-SDH mAbs. Peritoneal lavage was performed after 4 h and target cell number was analyzed by FACS. The proportion of survival CD20+ Daudi cells, determined by FACS using counting beads and normalized to control, is depicted in the graph, for the untreated mice (white, dotted pattern), or treatment with control NK-EVs (red, horizontal stripes), or RTX-SDH mAb armed NK-EVs (blue, right-leaning stripes). **Fig. 10B****.** At 48 h post fertilization, CTV-Daudi and CFSE-Nalm6 cells were co-injected into the brain ventricle of zebrafish larvae, and then treated with 10 µg of NK-EV unarmed (control) or armed with RTX-SDH mAbs. After 24h, larvae were anesthetized and dorsal imaging of tumor target cells was performed by confocal microscopy. The panel shows representative Z-stack projection of zebrafish with no treatment (top), or treated with control NK-EVs (middle), or treated with RTX-SDH mAb armed NK-EVs (bottom), showing the presence of CFSE-stained Nalm6 (green, left), CTV-stained Daudi (violet, middle), and merged picture from both (green and violet, right), scale bars = 100 µm. For A, bar graphs represent mean ± SEM, n = 3-5 mice per group. One-way ANOVA, * p<0.05; ** p<0.01; *** p<0.001.
**Figure 11** represents the stability of SDH-mAbs on CD56+CD16+ NK cells after arming at different pHs. **Fig. 11A****.** MFI using an Anti-RTX 1h after arming. UCB-expanded NK cells (eNK) were armed with SDH-RTX, wt-RTX (WT) or nothing (NT) for 1h at different pHs. After washing, cells were incubated in the same pH for 1 h. Then, cells were washed, put at pH 7.4 and the presence of RTX was unveiled with an anti-RTX by gating in the CD56+/CD16+ NK subset. **Fig. 11B****.** eNK were incubated with SDH-modified Trastuzumab at different pHs and treated as in Fig.11A. Trastuzumab presence at different time points was analyzed by using an anti-trastuzumab and gating on CD56+/CD16+ NK subset. Bars represent mean ± SEM. Statistical significance between conditions (n=8) was determined by two-way ANOVA (C-D). * p < 0.05; ** p < 0.01; *** p < 0.001; ns = not significant. Black line represents pHn = normal medium = 7.4 - 7.6. Grey line represents pHa = L-A = L-Lactic acid = 6.5.

### EXAMPLES

### Example 1: Material and Methods

### Cell lines

NKCD16 cells were cultured in RPMI 1640 media (Gibco) supplemented with 10% fetal bovine serum (FBS) and 100 UI/mL human IL-2 (Miltenyi Biotec). The B lymphoblast-like cell lines Raji and Daudi, the lymphocyte-like cell line Nalm6, and the lymphoblastoid B cell line PLH were cultured in RPMI 1640 media (Gibco) supplemented with 10% FBS. The solid tumor cell lines SKBR3 and BT-20 (breast cancer), 143B (osteosarcoma) and HCT116 (colorectal cancer) were culture in phenol-red free DMEM media supplemented with 10% FBS, 2 g/L D-glucose, 1% GlutaMAX^{™} and 1% sodium pyruvate (complete DMEM, cDMEM). Adherent cells were used for experiments at confluency of -80%. Cell line identity was confirmed by flow cytometry and were regularly tested for mycoplasma.

### UCBMC purification

UCB mononuclear cells (UCBMC) were collected from UCB units using Ficoll^{®} Paque Plus (Sigma) by density gradient centrifugation. Briefly, one volume of Ficoll^{®} Paque Plus were added to conical tubes, and two volumes of blood (previously diluted 1:1 with RPMI media) were slowly deposited at the top. Tubes were centrifuged at 425 × g for 30 min at room temperature, without brake. Mononuclear cells were collected from buffy coat layer, washed in RPMI and resuspended in RPMI media supplemented with 10% FBS (Fig. 1).

### Enrichment, activation and expansion of human NK cells

Expanded NK (eNK) cells were expanded using two different protocols. UCB NK cells, due to their need for both KIR and KAR signals to reach a mature phenotype, were cultured with PLH, an EBV-transformed HLA-I+ B lymphoblastoid cell line which works as an accessory cell with both required signals. Conversely, PB NKs are fully mature and need only activating signals thus the EBV+ HLA-I negative cell line 721.221 was employed. As a first step, T cells and NKT cells were depleted from the cultures using anti-CD3 mAb, to favor NK cell expansion. Each protocol required different ratios of accessory cells, while IL-2 and IL-15 were added at the same concentrations. UCB NKs were treated with accessory cells and cytokines every 3 d and benefited from little manipulation. PB NKs were sustained for 5-6 d before culture renewal and withstood daily manipulation. By d 20, almost all cells from both sources present in the culture were CD56+ CD3- NK cells. The purity of NK cells was 94.28 ± 2.08% for UCB and 95.8 ± 1.46% as an average. The CD3+ fraction, which was successfully depleted at d 0, did not overtake the CD56+ population, allowing for successful expansion of NK cells. Beginning with 1E6 NKs in each expansion, PB NKs reached an average of 240E6 cells by d 20 and UCB NKs averaged a 700-fold expansion.

### Flow cytometry analysis

In brief, UCB-expanded NK cells or NKCD16 cells (2 × 105) were stained for surface markers with the following fluorochrome conjugated antibodies: CD3-APC, CD56-PE-Vio770, CD16-VioBlue, CD45-VioGreen, CD69-PE, NKp46-PE, NKp30-APC-Vio770 and NKp44 APC (all from Miltenyi Biotec). Solid tumor cells were detached and stained with EGFR-PE or IgG2b (isotype, all from BD). Cell viability was determined using 7-AAD exclusion (Miltenyi Biotec). Staining was performed in FACS buffer at 4°C for 25-30 min, and then cells were washed three times before FACS acquisition and analysis in Gallios flow cytometer instrument (Beckman Coulter). After exclusion of doublets and dead cells, immune marker detection on CD56+ CD3- NK cells was evaluated by analyzing FCS files using FlowJo software v10.6.1 (Tree Star Inc.).

### Isolation of NK cell-derived extracellular vesicles (NK-EVs)

For isolation of NK-EVs, NKCD16 or UCB-derived eNK cells were washed and cultured for 48 h in EV-free RPMI medium 10% FBS (RPMI 10% FBS media ultracentrifugated at 100,000 × g for 18h at 4°C, then 0.22 µm filter-sterile). In order to obtain antibody-armed NK-EVs, NKCD16 or eNK cells were previously washed two times in plain RPMI 1640 media and incubated with SDH-modified (DFTE 26) CTX (anti-EGFR) or SDH-modified RTX (anti-CD20) mAbs at 10 µg/mL at a cell density of 10 × 106 cells/mL for 1 h at 37°C. In some experiments, after this incubation, cells were washed and labeled with CellTracker^{™} CFSE (Invitrogen) diluted in plain RPMI 1640 medium (1:3000) for 20 minutes at room temperature. After incubation, cells were washed two times with PBS buffer and resuspended in EV-free RPMI media 10% FBS at a density of 1x106/mL for NK-EVs obtention.

After 48 h incubation, cells were resuspended and transferred to 50 mL Falcon tubes, then cells and cell debris was removed from the conditioned media (CM) by serial centrifugation at 300 × g for 5 min, then 2,000 × g for 20 min. NK-EVs were recovered after ultracentrifugation at 100,000 × g for 120 min at 4°C, followed by washing with cold PBS and another step of ultracentrifugation at 100,000 × g for 120 min at 4°C. NK-EV pellets obtained by this method were resuspended in -100-200 µL PBS buffer and aliquoted for storage at -80°C until further use. To perform protein quantification, sample aliquots were first lysed by adding a tenth volume of 10X RIPA lysis buffer and incubating on ice for 20 min. After NK-EV lysis, protein quantification was performed by using the Micro BCA^{™} Protein Assay Kit (ThermoScientific), following manufacturer's instructions. Compared to tightly bound complexes, low-affinity protein associations are more sensitive to variations in their environment and rapidly respond to changes in temperature, pH, and solvent composition. It has been discussed that low-affinity interactions may be disrupted during ultracentrifugation (Holfeld A, et al. Mol Syst Biol. 2024. Ming Exp Biol Med 2019). The biochemical sensitivity of weak interactions is important for their function and enables them to fine-tune processes such as receptor signal transduction and stress adaptation mechanisms. Low-affinity interactions have also been well-characterized for their roles in different processes, such as controlling receptor-ligand promiscuity, or immune discrimination between "self" and "non-self", which recognize non-self molecules present in pathogens, but not present in the host. The interaction between the receptor FCγRIIIa/CD16a and the IgG Fc domain is a low-affinity protein-protein interaction. Of note, even if SDH-mAbs show increased affinity for CD16a than wt mAbs, their affinity it is still lower than that of an antibody for its antigen. It was unknown and unpredicted that the SDH-mAb stayed bound to CD16a after the NK-EV production, considering that other proteins do not survive this process, such as anti-CD56, presumably due to steps like ultracentrifugation. Thus, it was totally unexpected to recover SDH-mAb armed NK-EVs after 2 ultracentrifugation steps at 100,000 × g for 120 min.

### Nanoparticle Tracking Analysis (NTA)

NK-EVs were characterized by NTA using a NanoSight NS300 (Malvern). For the analyses, aliquots of NK-EV samples were diluted in PBS at a dilution of 1:250, before the capture of five videos of 60 seconds at a controlled temperature of 25°C. Video recordings from the samples were analyzed under pump-assisted constant flow (flow rate = 40). The recorded videos were analyzed to determine particle size and concentration with the NTA Software (Malvern), by setting a detection threshold of 5.

### NK-EVs cytotoxicity and binding assays on suspension tumor cells

The potency assay to determine cytotoxic function of NK-EVs on suspension cells was performed by FACS. Target cells from Raji and Daudi cell lines (CD20+) or Nalm6 (CD20-) were labeled with CellTrace^{™} CFSE (1:3000) diluted in plain RPMI for 20 min at room temperature and washed two times with RPMI 10% FBS, then resuspended in EV-free RPMI 10% FBS at cell concentration of 1x106/mL, and 100,000 target cells per well were seeded in round-bottom 96-well culture plates. These cells were incubated alone or with soluble RTX-SDH (anti-CD20) or CTX-SDH mAbs at 10 µg/mL, or in the presence of eNK cells at different effector-to-target ratios, as a control for NK cytotoxicity and ADCC. In addition, some cells were treated with PBS buffer (vehicle) or with NK-EVs from control (no mAb arming) or from RTX-SDH armed NK, or CTX-SDH armed NK at different doses (5 - 20 µg total protein), and then target cells were incubated 37°C, 5% CO₂ for 24 h. After incubation, cells were washed two times with PBS buffer before staining with CD56-APC (1:200) and Fixable Viability Dye eFluor^{™}780 (1:1000) for 20-30 min at 4°C. Then cells were washed two times and resuspended in PBS 2% FBS, 1 mM EDTA before FACS analysis on FACSCanto II cytometer and FACS data was analyzed using FlowJo v10.

### Direct arming of NK-derived EVs with mAbs

NK-EVs were functionally characterized to determine the capacity to "be armed" with mAbs after NK cell secretion, isolation and storage at -80°C. NK-derived EVs of known concentration were thawed on ice for 30 min, and aliquots of NK-EVs were diluted in PBS buffer up to a concentration of 100 µg/mL. Then, EVs were incubated with soluble wt RTX or SDH-modified (DFTE) RTX (both anti-CD20), or SDH-modified CTX (anti-EGFR) mAbs at 10 µg/mL in PBS buffer for 1 h at 37°C. After mAb arming, EVs were washed with PBS and ultracentrifuged at 100.000 × g for 120 min. EV pellets obtained by this method were resuspended in 100 µL of complete RPMI culture media and used the same day. The potency assay to determine cytotoxic function of these mAb-armed NK-EVs on suspension tumor cells was perform as mentioned before.

### NK-EVs binding assays on solid tumor cells

The potency assays to determine NK-EVs binding to adherent target cells was performed by fluorescence microscopy. Flat-bottom, µClear black-wall 96-well culture plates (Greiner) were pre-coated with poly-D-lysine (PDL, 20 µg/mL) for 1 h at 37°C, and washed with PBS before use. Solid tumor target cells MDA-MB-468 at 80% confluence were detached using TryPLE^{™} Express solution and washed with RPMI 10% FBS. After cell count, viability was determined and target cells were resuspended in plain RPMI 1640 medium containing CellTrace^{™} Far Red (CTFR, 1:3000) and incubated for 20 min at room temperature. Cells were then washed two times with RPMI 10% FBS, then resuspended in RPMI 10% FBS at cell concentration of 1x106/mL, and 50,000 target cells per well were seeded into PDL-coated plate wells and incubated overnight at 37°C. After removing media, cell plates were washed with PBS buffer and EV-free RPMI 10% FBS media was placed in all wells. These cells were incubated alone, or with PBS buffer (vehicle) or with 20 µg of CFSE-stained, NK-derived EVs from control conditions (no mAb arming) or from RTX-SDH arming, or CTX-SDH arming. Cells were incubated at 37°C, 5% CO₂ for 3 h. After washing plate wells, cells were fixed with 4% PFA solution (Sigma) for 15 min at 4°C, washed again and stained for nucleus with Hoescht (1:2000) diluted in PBS for 5 min. After two more washing steps with PBS, cell micrographs were obtained in a SP5 fluorescence microscope (Leica).

### Cytotoxicity assays of NK-EVs on solid tumor cells

The potency assay to determine cytotoxic function of NK-EVs on adherent tumor cells was performed by MTT viability assay. MDA-MB-468 (EGFR+) were detached and resuspended in RPMI 10% FBS medium, and 50,000 cells per well were seeded in PDL-coated 48-well plates and incubated overnight at 37°C. Cells were then washed with PBS buffer and media was replaced with EV-free RPMI 10% FBS. These cells were incubated alone or with soluble CTX-SDH at 10 µg/mL, or in the presence eNK cells at different effector-to-target ratios, as a control for NK cytotoxicity and ADCC. In addition, some cells were treated with PBS buffer (vehicle) or with NK-EVs from control conditions (no mAb arming) or from RTX-SDH arming, or CTX-SDH arming at different EV doses (10 - 40 µg total protein). Cells were incubated at 37°C, 5% CO2 for 24 h. After incubation, cells were washed two times with PBS 2% FBS 1 mM EDTA to wash away NK cells, and target cells were incubated with RPMI 10% FBS supplemented with 0.5 mg/mL of MTT (3-(4,5-dimethylthiazol-2-yl)-2,5-diphenyltetrazolium bromide, Sigma) for 20 min at 37°C to achieve formazan compound formation. Media was removed from cells, formazan in each well was solubilized with 1 N isopropanol, and plate wells were measured for absorbance at 560 nm and 655 nm (reference) in Varioskan Flash (ThermoScientific).

### Cytotoxicity assays of NK-EVs on solid tumor spheroids

The potency assays to determine NK-EV cytotoxicity to solid tumor spheroids were performed by kinetic imaging and cellular analysis of 3D targets. Solid tumor cell lines SKBR3 and BT, 143B and HCT116 were seeded into Nunclon Sphera round-bottom 96-well plate (ThermoFisher Scientific) in 200 µL of cDMEM medium. After 72 h, media was replaced with fresh media containing 5 µM CellEvent^{™} Caspase-3/7 Green Detection Reagent (ThermoFisher Scientific), and spheroids were treated in duplicate with PBS (vehicle) or 20 µg of NK-EVs from conditions with no arming (no mAb), CTX-SDH arming or RTX-SDH arming. Spheroids were monitored by kinetic imaging on bright field and fluorescence microscopy (laser 469 nm) in a Cytation 5 instrument (Biotek-Agilent). Spheroid size and green fluorescence (representing apoptosis) were measured each two hours for up to 5 days; captured images were pre-processed to remove background signal, change of spheroid size and mean spheroid green fluorescence (filter 525 nm) over time was analyzed by using the Cellular Analysis tool of Cytation 5 software Gen 5.3 (Biotek-Agilent).

### In vivo mice cytotoxic experiments

NOD/LtSz-SCID/IL-2Rγchain null (NSG) mice were produced at the Institute of Molecular Genentic of Montpellier (IGMM) Animal Facility (Montpellier-France) using breeders coming from Charles River Laboratories.

8-to-10 weeks-old female and male NSG mice received intraperitoneal injection of Daudi cells (pre-stained with 1 µM CellTrace^{™} Violet or CTV) and Nalm6 cells (pre-stained with 1 µM CFSE), along with unarmed NK-EVs or RTX-SDH-armed NK-EVs. After four hours, mice were euthanized and cells harvested by intraperitoneal lavage with 3 mL cold PBS containing 3% FBS. Red blood cells were removed using RBC Lysis Buffer (Biolegend), and murine FcγR were blocked with FcBlock (eBiosciences). Cells were stained with anti-human CD56-APC (Miltenyi), anti-mouse CD45-BV650 (eBiosciences) and Fixable Viability Dye eFluor^{™}780. After washing, cells were analyzed in a Cytek Aurora spectral flow cytometer using Precision Counting Beads (Biolegend).

### In vivo zebrafish cytotoxic experiments

Zebrafish larvae were obtained by natural spawning from pairs or adults then raised at 28.5°C. The zebrafish lines used were as follows: AB wild type zebrafish (ZIRC). Brain ventricle injections were performed in 48 hours post-fecundation (hpf) larvae anaesthetized with 0.016% Tricaine (Sigma) diluted in zebrafish water and mounted ventral side up in 1.5% low melting point agarose (LMP agarose, Sigma). Larvae received Daudi cells (pre-stained with 1 µM CTV) and Nalm6 cells (pre-stained with 1 µM CFSE) along with unarmed NK-EVs or RTX-SDH-armed NK-EVs. After 24 h, confocal imaging of the brain ventricles in live anesthetized larvae was done by acquisition of Z-stack series on Leica TCS SP8 inverted confocal microscope. Analysis and maximal intensity projections were generated on imaged.

### Statistical analysis

Experimental figures and statistical analysis were performed using GraphPad Prism (v8.0), for Student t-Test or One-way ANOVA. All statistical values are presented as * p<0.05; ** p<0.01; *** p<0.001. Mean values are expressed as mean plus or minus the standard error of the mean (SEM).

### Example 2: Obtention and characterization of NK-derived EVs

We used CD3-depleted UCBMCs to produce activated and expanded NK cells (eNK). We obtained a population of -98% CD56+ CD3- NK cells with high levels of CD16 expression. These eNK expressed high levels of the activation marker CD69 and the natural cytotoxicity receptors (NCR) NKp30, NKp44 and NKp46.NK cell source did not express NKp46. Cells were armed or not with SDH-modified mAbs, and then cultured in EV-free medium, in order to obtain only EVs derived from cultured NK cells (Fig. 3A). After 48 h of incubation, we cleared by serial centrifugation the conditioned media (CM) from these cultures and obtained NK-derived EVs by ultracentrifugation. Nanoparticle tracking analysis of NK-EV isolated samples showed a median size between 100-150 nm, most of them being in the range of 70-250 nm (Fig. 3B). Interestingly, in comparison with NK-EVs from control, without mAb arming, condition, NK-EVs from CTX-SDH and RTX-SDH presented a slight size increase of around 30 nm. eNK seem to secrete a high amounts of EV particles (-3x108 particles/106 cells) although protein quantification shows than the amount of EVs does not match the amount of EV particles (Fig. 3C), NK cells capacity to secrete EVs appeared not to be impaired by SDH-modified mAb-arming, due to similar EV yield obtained from the different conditions of about ~1 µg per million cultured cells (Fig. 3C). These results suggest that NK cells are able to secrete EVs under this culture conditions, and mAb-arming of these NKs do not affect EV yield and output.

Compared to tightly bound complexes, low-affinity protein associations are more sensitive to variations in their environment and rapidly respond to changes in temperature, pH, and solvent composition. It has been discussed that low-affinity interactions may be disrupted during ultracentrifugation (Holfeld A, et al. Mol Syst Biol. 2024. Ming, et al. Exp Biol Med. 2019). The biochemical sensitivity of weak interactions is important for their function and enables them to fine-tune processes such as receptor signal transduction and stress adaptation mechanisms. Low-affinity interactions have also been well-characterized for their roles in different processes, such as controlling receptor-ligand promiscuity, or immune discrimination between "self" and "non-self", which recognize non-self molecules present in pathogens, but not present in the host. The interaction between the receptor FcγRllla/CD16a and the IgG Fc domain is a low-affinity protein-protein interaction. Of note, even if SDH-mAbs show increased affinity for CD16a than Wt mAbs, their affinity it is still lower than that of an antibody for its antigen. It was unknown and unpredicted that the SDH-mAb stayed bound to CD16a after the NK-EV production, considering that other proteins do not survive this process, such as anti-CD56, presumably due to steps like ultracentrifugation. Thus, it was totally unexpected to recover SDH-mAb armed NK-EVs after 2 ultracentrifugation steps at 100,000 × g for 120 min.

Quantification experiments of NK-EVs secreted by unarmed controls or NK cells armed with mAbs showed no significant difference between the two groups, inferring that mAb arming of NK cells did not affect EVs production. This is an unexpected result, considering that the presence of mAb bound to CD16 receptors could have well impaired each step in the formation of early or late endosomes, as well as the biogenesis of small EVs in the interior of the multivesicular bodies, and ultimately impeding the obtention of mAb-armed NK-EVs from NK cells.

While performing the method for obtaining armed-EVs and after the step of incubating the NK cells with the antibody, the CD16/SDH-mAb complex first reaches the cytosol inside of an endosome. An invagination of the plasma membrane forms a cup- shaped structure that includes cell-surface proteins and soluble proteins associated with the extracellular milieu leading to the formation of an early-sorting endosome (Kalluri et al. Science, 2020). The subsequent interactions of the endosome with other intracellular vesicles and organelles generate the final content of the exosomes before being release out of the cell. It is surprising/unexpected that the low increase in affinity of SDH vs wt allows the CD16/SDH-mAb to perform all this travel through the EV biogenesis without destruction or loss of interaction. The endosome lumen pH turns very acidic through this process, under pH6 in some cases, creating a favorable environment for low-affinity interactions between proteins to be disrupted. Additionally, interaction with other proteins/enzymes could affect CD16a/SDH-mAb binding and/or structure during the process of endocytosis, endosome formation and release.

It was also unpredicted that NK-EVs bound to SDH-mAb and generated from the endocytic pathway can be released to the extracellular fluid compartment, considering that the endosomes that originate these EVs can also derive into lysosomes and autophagosomes. The content of these two vesicles are destinated to degradation and they will never be released to the extracellular compartment. In fact, some proteins that participates in EV biogenesis (such as Alix and Tsg101) are also part of the sorting process for lysosomal degradation. After mAb-arming of the NK cells, these SDH-Ab/CD16 complexes could have been subjected to lysosomal degradation after endocytosis, a process that commonly occurs to other cell receptors (Zastrow Annu Rev Biochem 2021). In summary, the binding of SDH-mAbs to NK-EVs or the stability of CD16/SDH-mAb binding after passing through endosomes is not predictable based on current literature.

### Example 3: Functional activity of mAb-armed NK-EVs on target cells

After confirming the nature of NK-EVs, their cytotoxic function on the CD20-expressing B-lymphoblastoid cell line Raji was analyzed. In the absence of mAbs, control NK-EVs (no mAb) induced a minor increase in cell death after 24 h of treatment, even at high NK-to-target ratio and the highest EV dose (Fig. 6A). In the presence of the anti-CD20 RTX-SDH, NK cells performed ADCC and robustly increased Raji cells apoptosis (Fig. 6B). Correspondingly, NK-EVs derived from RTX-SDH-armed NK cells significantly increased the frequency of dead cells in a dose-dependent manner (Fig. 6C). These results suggest that mAbs coming from the NK cell arming step are present in these NK-EVs, and the Ag-specific mAbs can increase the cytotoxic capacity of NK-EVs against targets cells.

Using the same strategy, these observations were extended by including two additional cell targets: the CD20+ B-lymphoblastic cancer cell line Daudi, and the CD20-negative B-cell precursor leukemia cell line Nalm6 (Fig. 6D). Consistent with the previous observations, "RTX-SDH-armed" NK-EVs increased Raji and Daudi death cell frequency in comparison with control unarmed NK-EVs (Fig. 6D-E). However, it was not the case when using Nalm6 as target cells, for which it was observed no difference between NK-EVs with or without mAbs. Taking into account the possibility that mAb arming process on parental NK cells could be modifying the composition of NK-EVs to increase their cytotoxic function independently of the mAb specificity, the function of NK-EVs armed with CTX-SDH (anti-EGFR) was also tested, a modified mAb with irrelevant specificity in relation with these hematological cancer cell lines. NK-EVs "armed" with CTX-SDH failed to increase tumor cell killing compared to unarmed control NK-EVs (Fig. 6D-E). These results suggest that parental NK cell arming with mAbs confers increased cytotoxicity to NK-derived EVs exclusively against targets expressing the antigen (Ag) recognized by the mAb.

NK-EVs can interact and be internalized by target cells via different mechanisms, such as endocytosis, fusion with the plasma membrane and receptor-ligand interaction. To examine the extent of target cells captured by NK-EVs, hematological cancer cells were treated with NK-EVs and CD56 surface expression was FACS analyzed, which expression has been reported present in NK-EVs. CD56 expression on these target cells was negligible, as observed under non-treated conditions or in presence of RTX-SDH mAb alone. When Raji cells were co-cultured with unarmed NK cells or treated with control NK-EVs, their CD56 surface detection was comparable to untreated conditions (Fig. 7A). However, there was a considerable (16-fold) increase in surface CD56 on Raji cells when treated with 20 µg of RTX-SDH-armed NK-EVs (Fig. 7A-B). This observation correlated with the cytotoxic effect of these mAb-armed NK-EVs, same effect with Daudi cells treated with RTX-SDH NK-EVs (20-fold increase) was observed. Treatment with unarmed control NK-EVs, or CTX-SDH armed NK-EVs also modestly increased Raji and Daudi surface CD56 (Fig. 7C-D). As anticipated for CD20-negative Nalm6 cells, the treatment with NK-EVs marginally changed surface CD56 expression detected by FACS, independently of the presence of mAbs on these EVs (Fig. 7C and E). This increase of NK CD56 on lymphoblastic targets suggests that Ag-specific mAbs present on NK-EVs favors their binding and capture by Ag-expressing target cells.

Subsequently, the binding potential of these armed NK-EVs to solid tumor cells was tested, as adherent target binding and capture of NK-EVs could be different than of hematological cancer cells in suspension. Breast cancer MDA-MB-468 target cells (expressing high levels of EGFR) were cultured and treated them with CellTrace^{™} CFSE-stained NK-EVs control or armed with CTX-SDH (anti-EGFR) mAbs for 3 h, then CFSE fluorescent signal by confocal microscopy was analyzed. Resembling to previously obtained results, CTX-SDH-armed NK-EVs seemed to better bind to target cells, showing higher CFSE signal around these cells, as compared to the unarmed NK-EV treated condition. Taken all together, these results support the notion that NK-EVs binding and capture by target cells can be enhanced by arming with SDH-modified mAbs, which positively impacts the immune function of these EVs.

### Example 4: CTX-SDH-armed NK-EVs induce apoptosis on tumor spheroids

After confirming that these NK-EVs successfully bind to solid tumor cells, it was compared their cytotoxic function against eNK cells. This was accomplished by first treating MDA-MB-468 cells and then by testing target cell viability using a MTT assay at 24 h. Basal killing capacity (natural cytotoxicity) of UCB-derived eNK cells was better than of NKCD16 cells, although both NK cell types are highly cytotoxic when in presence of CTX-SDH mAbs. Conversely, target killing levels mediated by eNK-EVs was lower in comparison to their parental NK cells, but increasing in a dose dependent manner, reaching similar cytotoxicity levels at 40 µg dose. NK-EVs from CTX-SDH-armed NK cells did not show improved cytotoxicity compared to those from non-armed NK cells.

In order to further elucidate the function of these mAb-armed NK-EVs on solid tumor cells, an alternative approach with better physiological relevance was taken, such as the use of 3D tumor cell spheroids. Spheroids were generated from different cell lines, such as 143B, HCT116, SKBR3 and BT-20. These spheroids had all different expression levels of EGFR depending on the cell line of origin. As a way to measure tumor cell death, the induction of apoptosis by GFP expression and changes in spheroid size were checked (Fig. 9A).

It was observed that NK-EV treatment increased caspase-3/7 activity in treated SKBR3 spheroids, in comparison with vehicle (PBS) and with untreated conditions, and the induced apoptosis by CTX-SDH-armed NK-EVs was faster and at higher levels than by unarmed NK-EVs or armed with an irrelevant mAb, i.e. RTX-SDH (Fig. 9B). As expected, this effect correlated with less growth on spheroids treated with CTX-SDH-armed NK-EVs, which supported the notion that mAb specificity is relevant for NK-EV binding and function (Fig. 9C).

Similar results were obtained for spheroids from BT-20 and 143B cell lines, with high and medium expression levels of EGFR, respectively (Fig. 9). Interestingly, this treatment was less effective on spheroids composed of HCT116 colorectal cancer cells, which express lower levels of EGFR, for which treatments with NK-EVs or vehicle showed no apparent differences in caspase-3/7 activity or in spheroid growth (Fig. 9). Taken together, these results provide evidence for the positive role of mAbs present in EVs from mAb-armed NK cells on the cytotoxic function of these NK-EVs, and this support the use of these mAb-armed NK-EVs as potential candidates for cancer patient treatment.

### Example 5: In vivo cytotoxicity of SDH-RTX-armed NK-EVs

The Authors investigated the cytotoxic activity of SDH-RTX NK-EVs in two in vivo models. In the first experiment, Daudi cells were intraperitoneally injected into NSG mice (Fig. 10A), mice were then randomly divided into three groups, each receiving a different treatment approach: mice in the control group received no treatment, mice in the negative control group received a treatment with unarmed NK-EVs, and mice in the experimental group received the SDH-RTX NK-EVs treatment. Although the NK-EVs showed a strong tumoricidal activity, the SDH-RTX NK-EVs treatment was significantly more efficacious showing a statistically significant increase in cytotoxic effects on Daudi cells. In the second experiment, Daudi cells and Nalm6 cells were co-injected into the brain ventricle of zebrafish larvae (Fig. 10B), fish were then randomly divided into three groups, each receiving a different treatment approach: fish in the control group received no treatment, fish in the negative control group received a treatment with unarmed NK-EVs, and fish in the experimental group received the SDH-RTX NK-EVs treatment. Whereas NK-EV treatment decreased equally both Daudi cells and Nalm6 cells, SDH-RTX NK-EV treatment kept it specificity showing a cytotoxicity directed against Daudi cells. These results show that NK-EVs armed with an antibody are able to keep their specificity also in vivo, resulting in a stronger effect and more directed cytotoxic efficiency.

### Example 6: stability of the SDH-mAb-CD16 complex through acidic PHs

We incubated NK cells at different pHs with wt-RTX or SDH-RTX or nothing (NT) for 1h, washed and incubated for 1h in the same pH. Next, cells were incubated with an anti-RTX and the amount of wt-RTX or SDH-RTX in the cells was analyzed by flow cytometry in the CD56+CD16+ population (Fig. 11A). Unexpectedly, this figure showed that acidic pH at the time of arming increases the stability of SDH-modified mAbs on the CD16 of NK cells. We next further this result by showing that SDH-trastuzumab was also more stable on NK cells when arming was performed at acidic pH (Fig. 11B). The improved stability was maintained for at least 72 h. Therefore, by an unknown mechanism acidic pH favors the stability of SDH-Fc molecules on the CD16 of NK cells.

### Sequence Listings

SEQ ID NO. 1 (=Fc WT region (lgG1-CH 2)) 
APELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAK
SEQ ID NO. 2 (=Fc SD region (lgG1-CH2 S239D)) 
APELLGGPDVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAK
SEQ ID NO. 3 (=Fc IE region (lgG1-CH2 I332E)) 
APELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPEEKTISKAK
SEQ ID NO. 4 (=Fc HF region (lgG1-CH2 H268F)) 
APELLGGPSVFLFPPKPKDTLMISRTPEVTCVVVDVSFEDPEVKFNWYVDGVEVHNA
KTKPREEQYNSTYRVVSVLTVLHQDWLNGKEYKCKVSNKALPAPIEKTISKAK
SEQ ID NO. 5 (=Fc ST region (lgG1-CH2 S324T)) 
APELLGGPSVFLFPPKPKDTLMISRTPEVTCVVVDVSHEDPEVKFNWYVDGVEVHNA
KTKPREEQYNSTYRVVSVLTVLHQDWLNGKEYKCKVTNKALPAPIEKTISKAK
SEQ ID NO. 6 (=Fc GA region (lgG1-CH2 G236A)) 
APELLAGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAK
SEQ ID NO. 7 (=Fc AL region (lgG1-CH2 A330L)) 
APELLGGPDVFLFPPKPKDTLMISRTPEVTCVVVDVSHEDPEVKFNWYVDGVEVHNA
KTKPREEQYNSTYRVVSVLTVLHQDWLNGKEYKCKVSNKALPLPIEKTISKAK
SEQ ID NO. 8 (=Fc SDH region (lgG1-CH 2.S239D.H268F.S324T.I332E))

APELLGGPDVFLFPPKPKDTLMISRTPEVTCVVVDVSFEDPEVKFNWYVDGVEVHNA
KTKPREEQYNSTYRVVSVLTVLHQDWLNGKEYKCKVTNKALPAPEEKTISKAK

### NON-PATENT REFERENCES:

Wu F, Xie M, Hun M, et al. Natural Killer Cell-Derived Extracellular Vesicles: Novel Players in Cancer Immunotherapy. Front Immunol. 2021 May 21;12:658698.
Wang X, Yang X, Wang Y, et al. Combination of Expanded Allogeneic NK Cells and T Cell-Based Immunotherapy Exert Enhanced Antitumor Effects. Cancers (Basel). 2022 Dec 30;15(1):251.
Sheta M, Taha EA, Lu Y, et al. Extracellular Vesicles: New Classification and Tumor Immunosuppression. Biology (Basel). 2023 Jan 10;12(1):110.
He S, Su L, Hu H, et al. Immunoregulatory functions and therapeutic potential of natural killer cell-derived extracellular vesicles in chronic diseases. Front Immunol. 2024 Jan 3;14:1328094.
Stenger TD, Miller JS. Therapeutic approaches to enhance natural killer cell cytotoxicity. Front Immunol. 2024 Mar 11;15:1356666.
Page A, Chuvin N, Valladeau-Guilemond J, et al. Development of NK cell-based cancer immunotherapies through receptor engineering. Cell Mol Immunol. 2024 Apr;21(4):315-331.
Lugini L, Cecchetti S, Huber V, et al. Immune surveillance properties of human NK cell-derived exosomes. J Immunol. 2012 Sep 15;189(6):2833-42.
Federici C, Shahaj E, Cecchetti S, et al. Natural-Killer-Derived Extracellular Vesicles: Immune Sensors and Interactors. Front Immunol. 2020 Mar 13;11:262.
Claridge B, Lozano J, Poh QH, et al. Development of Extracellular Vesicle Therapeutics: Challenges, Considerations, and Opportunities. Front Cell Dev Biol. 2021 Sep 20;9:734720.
Rafieezadeh D, Rafieezadeh A. Extracellular vesicles and their therapeutic applications: a review article (part1). Int J Physiol Pathophysiol Pharmacol. 2024 Feb 25;16(1):1-9. Qi Y, Zhao X, Dong Y, et al. Opportunities and challenges of natural killer cell-derived extracellular vesicles. Front Bioeng Biotechnol. 2023 Mar 31;11:1122585.
Chan AML, Cheah JM, Lokanathan Y, et al. Natural Killer Cell-Derived Extracellular Vesicles as a Promising Immunotherapeutic Strategy for Cancer: A Systematic Review. Int J Mol Sci. 2023 Feb 16;24(4):4026.
Meng F, Zhang S, Xie J, et al. Leveraging CD16 fusion receptors to remodel the immune response for enhancing anti-tumor immunotherapy in iPSC-derived NK cells. J Hematol Oncol. 2023 Jun 14;16(1):62.
**Martin Villalba,** Michaël Constantinides, Loïs Coënon et al. The arming of NK cells with Fc-engineered rituximab confers them specificity against CD20-expressing cells, 11 March 2024, PREPRINT available at *Research Square.* doi.org/10.21203/rs.3.rs-4017845/v1.
Davis RS, Dennis G Jr, Odom MR, et al. Fc receptor homologs: newest members of a remarkably diverse Fc receptor gene family. Immunol Rev. 2002 Dec;190:123-36.
Edelman GM, Cunningham BA, Gall WE, et al. The covalent structure of an entire gammaG immunoglobulin molecule. Proc Natl Acad Sci U S A. 1969 May;63(1):78-85.
Ward ES, Güssow D, Griffiths AD, et al. Binding activities of a repertoire of single immunoglobulin variable domains secreted from Escherichia coli. Nature. 1989 Oct 12;341(6242):544-6.
Bird RE, Hardman KD, Jacobson JW, et al. Single-chain antigen-binding proteins. Science. 1988 Oct 21;242(4877):423-6.
Huston JS, Levinson D, Mudgett-Hunter M, et al. Protein engineering of antibody binding sites: recovery of specific activity in an anti-digoxin single-chain Fv analogue produced in Escherichia coli. Proc Natl Acad Sci USA. 1988 Aug;85(16):5879-83.
Holliger P, Hudson PJ. Engineered antibody fragments and the rise of single domains. Nat Biotechnol. 2005 Sep;23(9):1126-36.
Zapata G, Ridgway JB, Mordenti J, et al. Engineering linear F(ab')2 fragments for efficient production in Escherichia coli and enhanced antiproliferative activity. Protein Eng. 1995 Oct;8(10):1057-62.
Kalluri R, LeBleu VS. The biology, function, and biomedical applications of exosomes. Science. 2020 Feb 7;367(6478):eaau6977.
von Zastrow M, Sorkin A. Mechanisms for Regulating and Organizing Receptor Signaling by Endocytosis. Annu Rev Biochem. 2021 Jun 20;90:709-737. doi: 10.1146/annurev-biochem-081820-092427.
Patel KR, Nott JD, Barb AW. Primary Human Natural Killer Cells Retain Proinflammatory IgG1 at the Cell Surface and Express CD16a Glycoforms with Donor-dependent Variability. Mol Cell Proteomics. 2019 Nov;18(11):2178-2190.
Holfeld A, Schuster D, Sesterhenn F, et al. Systematic identification of structure-specific protein-protein interactions. Mol Syst Biol. 2024 Jun;20(6):651-675.
Miernyk JA, Thelen JJ. Biochemical approaches for discovering protein-protein interactions. Plant J. 2008 Feb;53(4):597-609.
Ming Q, Gonzalez-Perez D, Luca VC. Molecular engineering strategies for visualizing low-affinity protein complexes. Exp Biol Med (Maywood). 2019 Dec;244(17): 1559-1567. Wang W, Antonsen K, Wang YJ, Wang DQ. pH dependent effect of glycosylation on protein stability. EurJ Pharm Sci. 2008 Feb 5;33(2):120-7.
**Coënon L,** Villalba M. From CD16a Biology to Antibody-Dependent Cell-Mediated Cytotoxicity Improvement. Front Immunol. 2022 Jun 3;13:913215.
**Coënon L,** Rigal E, Courot H, et al. Generation of non-genetically modified, CAR-like, NK cells. J Immunother Cancer. 2024 Jul 18;12(7):e009070.
Roberts JT, Barb AW. A single amino acid distorts the Fc γ receptor Illb/CD16b structure upon binding immunoglobulin G1 and reduces affinity relative to CD16a. J Biol Chem. 2018;293(51):19899-19908.
Wang Y, Wu J, Newton R, Bahaie NS, et al. ADAM17 cleaves CD16b (FcγRlllb) in human neutrophils. Biochim Biophys Acta. 2013 Mar;1833(3):680-5.
Wang W, Antonsen K, Wang YJ, Wang DQ. pH dependent effect of glycosylation on protein stability. Eur J Pharm Sci. 2008 Feb 5;33(2):120-7.
Breitling J, Aebi M. N-linked protein glycosylation in the endoplasmic reticulum. Cold Spring Harb Perspect Biol. 2013 Aug 1;5(8):a013359.
Miernyk JA, Thelen JJ. Biochemical approaches for discovering protein-protein interactions. Plant J. 2008 Feb;53(4):597-609.

## Claims

1. An extracellular vesicle (EV) derived from a CD16+ cell, complexed to at least one recombinant polypeptide capable of binding to a FcyRIII/CD16 surface protein, wherein the recombinant polypeptide is non-covalently bound to the FcyRIII/CD16 surface protein located on the vesicle, and wherein said recombinant polypeptide comprises:
(i) a modified CH2 domain of a wild-type human IgG1-represented by SEQ ID NO 1; wherein the modified CH2 domain comprises amino acid substitutions S239D and I332E with respect to the CH2 domain of a wild-type human IgG1; bound to
(ii) a ligand binding domain, wherein the ligand binding domain comprises a sequence capable of binding to a target ligand.

2. The EV according to claim 1, wherein the modified CH2 domain comprises at least one additional amino acid substitution selected from the group consisting of H268F, S324T, G236A, A330L, and any combination thereof.

3. The EV according to any one of claims 1 or 2, wherein the modified CH2 domain comprises amino acid substitutions S239D, I332E, H268F, and S324T with respect to the CH2 domain of a wild-type human IgG1.

4. The EV according to any one of claims 1 to 3, wherein the FcyRIII/CD16 surface protein is a FcyRllla/CD16a surface protein or a FcyRlllb/CD16b surface protein.

5. The EV according to any one of claims 1 to 4, wherein the extracellular vesicle is derived from a CD16+ cell that is allogeneic or autologous with respect to an individual in need thereof.

6. The EV according to any one of claims 1 to 5, wherein the modified CH2 domain is bound to the ligand binding domain through a linker.

7. The EV according to any one of claims 1 to 6, wherein the recombinant polypeptide comprises a human IgG1 Fc region comprising the modified CH2 domain.

8. The EV according to any one of claims 1 to 7, wherein the recombinant polypeptide is an antibody or a fragment thereof, and the modification in the CH2 domain is symmetrical, or asymmetrical, with respect to the pair of CH2 domains constituting the antibody.

9. The EV according to any one of the claims 1 to 8, wherein the CD16+ cell is a NK cell or a neutrophil.

10. A method of obtaining at least one extracellular vesicle according to any of claims 1 to 9, the method comprising:
(i) incubating the recombinant polypeptide with the CD16+ cell or with the extracellular vesicle derived from a CD16+ cell, for forming a complex;
and wherein, if the incubating step (i) is carried out with the CD16+ cell, the method further comprises:
(ii) collecting at least one extracellular vesicle complexed to the recombinant polypeptide.

11. The method of obtaining at least one extracellular vesicle according to claim 10, wherein extracellular vesicle derived from a CD16+ cell has been previously frozen.

12. The EV according to any of claims 1 to 9, for use in medicine.

13. The EV according to claim 12, for use in a method for treating or preventing one disease selected from the group consisting of: cancer, autoimmune disease, and an infectious disease in an individual in need thereof.

14. A composition comprising at least one EV defined in any one of the claims 1 to 9.

15. The composition according to claim 14, which is a pharmaceutical composition.

16. The pharmaceutical composition according to claim 15, wherein it comprises an excipient or a pharmacologically acceptable vehicle.
